(19) Europäisches Patentamt
European Patent Office
Office européen des brevets

(11) **EP 4 061 447 B1**

(12) **EUROPEAN PATENT SPECIFICATION**

(45) Date of publication and mention
of the grant of the patent:
**15.10.2025 Bulletin 2025/42**

(21) Application number: **20890831.9**

(22) Date of filing: **18.11.2020**

(51) International Patent Classification (IPC):
**A61M 1/28** (2006.01)

(52) Cooperative Patent Classification (CPC):
**A61M 1/281; A61M 1/282; A61M 1/285;**
A61M 2205/3334; A61M 2205/3344; A61M 2205/50

(86) International application number:
**PCT/US2020/061074**

(87) International publication number:
**WO 2021/102012 (27.05.2021 Gazette 2021/21)**

(54) **PERITONEAL DIALYSIS SYSTEMS, DEVICES, AND METHODS**

PERITONEALDIALYSESYSTEME, -VORRICHTUNGEN UND -VERFAHREN

SYSTÈMES, DISPOSITIFS ET PROCÉDÉS DE DIALYSE PÉRITONÉALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.11.2019 US 201962938429 P**

(43) Date of publication of application:
**28.09.2022 Bulletin 2022/39**

(73) Proprietor: **NxStage Medical, Inc.**
**Lawrence, MA 01843 (US)**

(72) Inventors:
• **WYETH, Mark T.**
**Andover, MA 01810 (US)**
• **MCCARTY, Robert Paul**
**Reading, MA 01867 (US)**
• **YANTZ, Gregory**
**Boxford, MA 01921 (US)**
• **BOWEN, Winston**
**Allston, MA 02134 (US)**

(74) Representative: **Freischem & Partner**
**Patentanwälte mbB**
**Salierring 47-53**
**50677 Köln (DE)**

(56) References cited:
**US-A1- 2013 228 505     US-A1- 2016 375 189
US-A1- 2017 203 026     US-A1- 2017 326 282
US-A1- 2019 262 526     US-B1- 6 497 676**

**Description**

Cross-Reference to Related Applications

This application claims priority to and the benefit of U.S. Provisional Patent Application No. 62/938,429 filed November 21, 2019.

Background

Peritoneal dialysis is a mature technology that has been in use for many years. It is one of two common forms of dialysis, the other being hemodialysis which uses an artificial membrane to directly cleanse the blood of a renal patient. Peritoneal dialysis employs the natural membrane of the peritoneum to permit the removal of excess water and toxins from the blood. In peritoneal dialysis, sterile peritoneal solution is infused into a patient's peritoneal cavity using a catheter that has been inserted through the abdominal wall. The solution remains in the peritoneal cavity for a dwell period. Osmosis exchange with the patient's blood occurs across the peritoneal membrane, removing urea and other toxins and excess water from the blood. Ions that need to be regulated are also exchanged across the membrane. The removal of excess water results in a higher volume of fluid being removed from the patient than is infused. The net excess is called ultrafiltrate, and the process of removal is called ultrafiltration. After the dwell time, the dialysate is removed from the body cavity through the catheter.

Peritoneal dialysis requires the maintenance of strict sterility because of the high risk of peritoneal infection. The risk of infection is particularly high due to the long periods of time that the patient is exposed to the dialysate. In one form of peritoneal dialysis, which is sometimes referred to as cycler-assisted peritoneal dialysis, an automated cycler is used to infuse and drain dialysate. This form of treatment can be done automatically at night while the patient sleeps. One of the safety mechanisms for such a treatment is the monitoring by the cycler of the quantity of ultrafiltrate. The cycler performs this monitoring function by measuring the amount of fluid infused and the amount removed to compute the net fluid removal. The treatment sequence usually begins with an initial drain cycle to empty the peritoneal cavity of spent dialysate, except on so-called "dry days" when the patient begins automated treatment without a peritoneum filled with dialysate. The cycler then performs a series of fill, dwell, and drain cycles, typically finishing with a fill cycle. The fill cycle presents a risk of over-pressurizing the peritoneal cavity, which has a low tolerance for excess pressure. In traditional peritoneal dialysis, a dialysate container is elevated to certain level above the patient's abdomen so that the fill pressure is determined by the height difference. Automated systems sometimes employ pumps that cannot generate a pressure beyond a certain level, but this system is not foolproof since a fluid column height can arise due to a patient-cycler level difference and cause an overpressure. A reverse height difference can also introduce an error in the fluid balance calculation because of incomplete draining.

Some cyclers fill by regulating fill volume during each cycle. The volume may be entered into a controller based on a prescription. The prescription, which also determines the composition of the dialysate, may be based upon the patient's size, weight, and other criteria. Due to errors, prescriptions may be incorrect or imperfectly implemented resulting in a detriment to patient well-being and health. Systems that measure pressure have been proposed. For example, a pressure sensor in contact with a fluid circuit at the cycler has been described. The pressure sensor indicates the pressure at the proximal end of the access line. During operation, a controller connected to the pressure sensor changes the operation of the peritoneal dialysis machine in response to changes in pressure sensed by the pressure sensor.

An example of an automated peritoneal dialysis system is described in published international patent application PCT/US2012/056781.

It is referred to US 2017/0203026 AI as prior art. It describes a peritoneal dialysis cycler comprising a controller configured to perform a closed-loop control of the pump connected to the patient line.

Summary

The invention is defined in the appended claims.

It concerns a peritoneal dialysis cycler (101, 400), comprising:

a pump (206, 840, 858) connected to a patient access line (112, 450,832);
at least one pressure sensor (102, 110, 413, 414, 834) connected to the patient access line (112, 450, 832) to measure pressure of fluid delivered into said patient access line (112, 450, 832) or drawn from it; and
a controller (116, 202, 410, 830) configured to control the pump (206, 840, 858) and to perform cycler-assisted peritoneal dialysis treatment including delivering fluid from a fluid component (106, 441, 850) through the patient access line (112, 450, 832), the controller (116, 202, 410, 830) performing closed-loop control of the pump (206, 840, 858) responsively to one or more pressure feedback signals from said at least one pressure sensor (102, 110, 413, 414, 834); characterized in that the feedback signal is an envelope of a signal from the at least one pressure sensor (102, 110, 413, 414, 834).

An automated peritoneal dialysis system provides various features including prescription-driven dialysis fluid preparation, use of containers of pre-mixed dialysis fluid, an integrated disposable fluid circuit, and sensor capabilities that allow accurate filling and draining control with safety margins. Features include a peritoneal fluid circuit with various sensors, and methods and devices for using the sensor signals for pressure regulation during the fill and drain cycles, compensation of fluid volume measurement for measured air, and detection of reduced peritoneal volume due to adhesions or constipation. Other features and embodiments are disclosed.

Brief Description of the Drawings

Embodiments will hereinafter be described in detail below with reference to the accompanying drawings, wherein like reference numerals represent like elements. The accompanying drawings have not necessarily been drawn to scale. Where applicable, some features may not be illustrated to assist in the description of underlying features.

Fig. 1 shows a peritoneal dialysis system with pressure sensors located at a patient and at a peritoneal dialysis cycler, according to embodiments of the disclosed subject matter.

Fig. 2A shows a pod-type pressure sensor, according to embodiments of the disclosed subject matter.

Fig. 2B shows a peritoneal dialysis tubing set with an integrated pressure sensor according to embodiments of the disclosed subject matter.

Figs. 3A-3C show threads of a procedure for monitoring access processes of a cycler using pressure sensors according to embodiments of the disclosed subject matter.

Fig. 4A shows a peritoneal dialysis fluid proportioner/cycler according to embodiments of the disclosed subject matter.

Fig. 4B shows the peritoneal dialysis fluid proportioner/cycler of Fig. 4A in a first phase of fluid preparation in which osmotic agent concentrate is added to a mixing container, according to embodiments of the disclosed subject matter.

Fig. 4C shows the peritoneal dialysis fluid proportioner/cycler of Fig. 4A in a second phase of fluid preparation in which a dialysis fluid precursor is obtained by diluting and mixing the contents of the mixing container, according to embodiments of the disclosed subject matter.

Fig. 4D shows the peritoneal dialysis fluid proportioner/cycler of Fig. 4A in a third phase of fluid preparation in which the peritoneal dialysis fluid precursor properties are verified, according to embodiments of the disclosed subject matter.

Fig. 4E shows the peritoneal dialysis fluid proportioner/cycler of Fig. 4A in a fourth phase of fluid preparation in which dialysis fluid precursor is further prepared by addition of electrolyte concentrate to the mixing container, according to embodiments of the disclosed subject matter.

Fig. 4F shows the peritoneal dialysis fluid proportioner/cycler of Fig. 4A in a fifth phase of fluid preparation in which end-use dialysis fluid is prepared by adjustment of the dilution of the mixing container contents, according to embodiments of the disclosed subject matter.

Fig. 4G shows the peritoneal dialysis fluid proportioner/cycler of Fig. 4A in a sixth phase of fluid preparation in which dialysis fluid in the mixing container is verified, according to embodiments of the disclosed subject matter.

Fig. 4H shows the peritoneal dialysis fluid proportioner/cycler of Fig. 4A in various peritoneal dialysis treatment modes, according to embodiments of the disclosed subject matter.

Fig. 4J shows a peritoneal dialysis fluid proportioner/cycler similar to that of Fig. 4A in which a single mixing container line connects a valve network to the mixing container.

Fig. 4K shows the peritoneal dialysis fluid proportioner/cycler of Fig. 4A in various peritoneal dialysis treatment modes, according to embodiments of the disclosed subject matter.

Fig. 5 illustrates a control system according to embodiments of the disclosed subject matter.

Fig. 6 shows a fluid path and actuator layout according to embodiments of the disclosed subject matter.

Fig. 7 shows a cross-sectional view of a fluid line with an ultrasound detector according to embodiments of the disclosed subject matter.

Fig. 8 shows a diagram of a control system for pumping control in a cycler, according to embodiments of the disclosed subject matter.

Fig. 9 shows a diagram of a closed-loop control system according to embodiments of the disclosed subject matter.

Figs. 10 and 11 show envelope follower and filter outputs according to embodiments of the disclosed subject matter.

Fig. 12 shows a block diagram of a computer system that is relevant to controllers.

Detailed Description

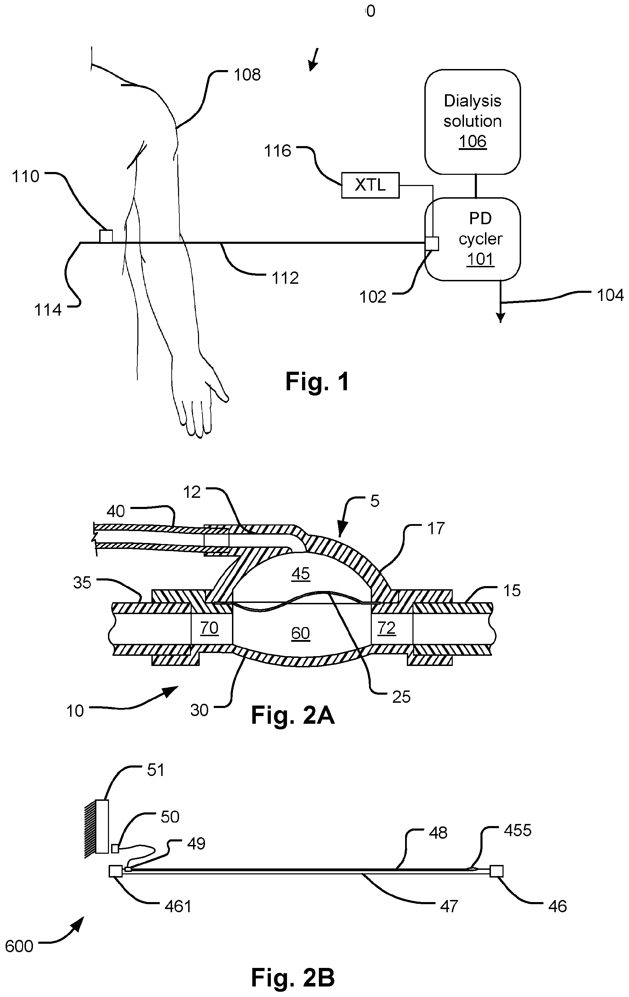

Referring to Fig. 1, a peritoneal dialysis system 100 includes a peritoneal dialysis (PD) cycler 101 with an internal pump (not shown). The PD cycler 101 pumps dialysis solution from a container 106, such as a bag or other source, through an access line 112 to a patient access 114 which is a peritoneal catheter that is inserted into the peritoneum of a patient 108. This happens during a fill cycle. During a drain cycle, spent dialysate is withdrawn from the patient by flowing in reverse through the access line back to the cycler 101 and out through a drain 104. The cycler 101 quantifies the volume of fluid that is infused and drained and provides an accounting of the difference to allow the net amount of fluid withdrawn from the patient to be determined. The pump may be any suitable pump such as a diaphragm pump or a peristaltic pump. Alternatively, the cycler may rely on other fluid conveyance systems such as an over or under-pressurized supply/sump container, gravity feed, or any other suitable mechanism. A controller 116 allows the system to regulate a flow rate to ensure the patient's peritoneal cavity is not over-pressurized. The flow regulation may be accomplished by changing a speed of a pump or by means of a variable flow restrictor or any suitable mechanism conforming to the requirements of the type of fluid conveyance system employed.

Prior art systems have prevented exceeding a safe limit on peritoneal pressure by a variety of mechanisms, including measuring pressure in the fill line using a pressure sensor located on the PD cycler and applying feedback control of the pump to ensure a limit is not exceeded. Another prior art device for preventing over-pressurization of the peritoneal cavity limits the total head pressure by employing a gravitational feed. Another prior art system employs a pressure detection device located at the end of a fill line, adjacent the patient or at the patient access itself, to take pressure readings close to the patient. By using pressure measurements from this location, the error in pressure measurement of the peritoneal cavity due to pressure loss in the fill line during filling of the cavity is eliminated. In this way, the flow rate can be controlled by a continuous feedback loop to maintain the cavity pressure below a desired safety threshold. Locating the pressure sensor close to the patient also eliminates another source of error which may arise from a level difference between the supply side of the fill line and the catheter end of the fill line. That is, if the cycler is located higher than the patient access, the gravitational head pressure of the fill line could cause a greater pressure than indicated by a pressure sensor located at the PD cycler which may not otherwise be accounted for, causing excessive pressure to be applied.

In the embodiment of Fig. 1, to provide accurate pressure indication, the pressure detection device 110 is located close to the patient 108 to maximize responsiveness to changes in the peritoneal cavity pressure and minimize the effect of pressure drop due to flow resistance. An electrical pressure transducer may be located at the end of the line. Alternatively, a pressure pod as described in US patent publication 2007/0179422 may be used. In an embodiment, a pressure transducer may be located at the controller or cycler as shown in Fig. 1 and also at the patient access 114 to measure the pressure of the peritoneal space without the signal bias produced by line pressure drop in the line 112.

Fig. 2A shows a pressure measurement pod 10, or simply pod 10 for short. In the pod 10, air chamber 45 is in communication with an and air line 40 that can be connected to a pressure transducer (not shown). Fluid flows through a fluid chamber 60 between an inlet line 35 connected to an inlet port 70 and out of the fluid chamber 60 through an outlet port 72 into an outlet line 15. The pressure of the fluid in the fluid chamber 60 displaces a diaphragm 25 until the air chamber 45 and fluid chamber 60 are at equilibrium, which is preferably the situation when the air chamber 45 and fluid chamber 60 are at equal pressure. The pod 10 is primarily made of two parts, a fluid-side shell 30 and an air-side shell 17, that, together, form an enclosure 5 that defines the fluid chamber 60 and air chamber 45. The ratio of the minimum to the maximum volume of the air chamber 45, including the volume of the air line 40 and air port 12, is proportional to the total pressure variation that can be measured by the transducer attached to the air line 40.

Fig. 2B shows a peritoneal dialysis tubing set 600 with an integrated pressure sensor 455 located at a distal end of a fill-drain line 47. The fill-drain line 47 may have one or two lumens for shared or separate fill and drain use, respectively. A pressure transducer 49 is in pressure communication with a lumen of the fill-drain line 47. If there are separate fill and drain lumens, each may carry its own pressure transducer 49 or only one, for example, the fill line, may carry the pressure transducer 49. The transducer 49 may be, for example, a strain gauge component that reacts to isotropic pressure (e.g., fully wetted and immersed) or it may be a strain gauge component built into the wall of an inline fluid conveying component. Other configurations are also possible to achieve the effect of providing pressure sensing at the distal end of the fill-drain line 47. A pair (or more, as necessary) of conductors 48 may run along the length of the fill-drain line 47 to connect to an electrical connector 50 which connects to a driver circuit 51. The driver circuit may contain a power supply and reader circuit or other suitable circuitry for generating a pressure signal from the pressure applied by fluid in the lumen of the fill-drain line 47 at its distal end.

A connector 46 configured for connection to a peritoneal catheter is attached to the distal end and a connector 461 for connection to a source and/or sink of fluid is located on the proximal end of the fill-drain line 47. The connector 46 may be permanently attached to a peritoneal catheter or may have a peritoneal catheter preinstalled thereat. The connectors 46 and 461 may be sealed to isolate the lumen and the unit 600 delivered as a sealed unit with a sterile lumen which has been preconnected to a peritoneal catheter at a distal and to a treatment fluid circuit at the other end thereby defining a sterile barrier with the need to make connections after unpacking.

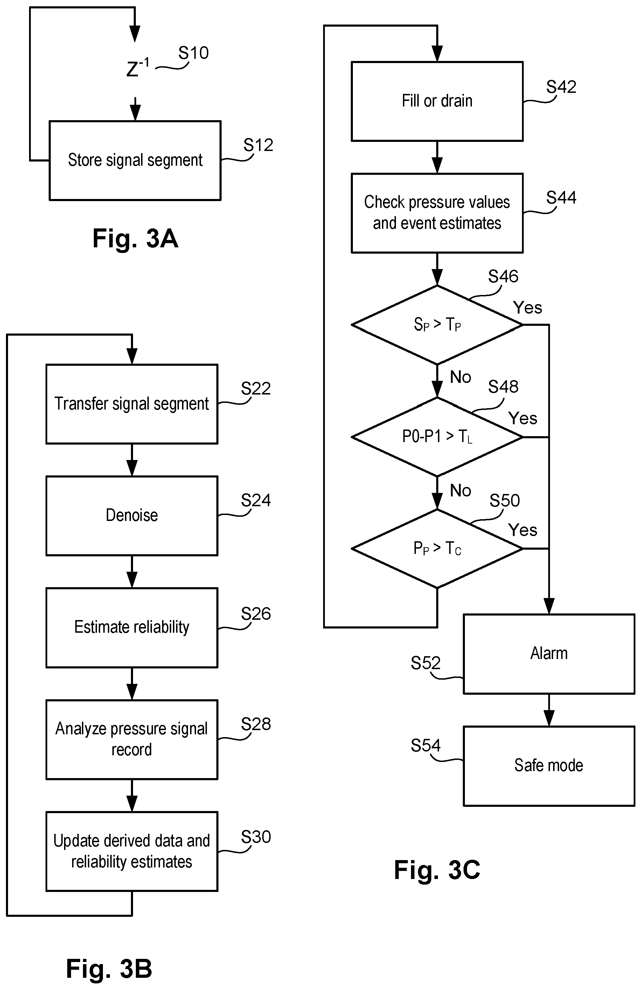

Referring now to Figs. 3A to 3C, an example process for monitoring pressure signals from the foregoing peritoneal devices is now described. Fig. 3A shows a process for storing a string of pressure signal samples for an interval of time. For example, the pressure signal may be sampled at 100 ms intervals for a period of 20 seconds at S12 and the process is repeated after a delay S10. The samples may be stored in a memory for many samples covering an entire treatment or for only a portion of a treatment. Alternatively, pressure data samples respective of each pressure sensor may be continuously stored in a memory and refreshed after archiving following a treatment or refreshed in a first-in first-out fashion according to a time interval so as to preserve only a short-term historical record. In another alternative, only instantaneous pressure data may be stored.

The procedure of Fig. 3B derives various information from the data stored by the operation of Fig. 3A. The operation may be applied to each pressure signal, including, for example, those provided by a distal pressure sensor (e.g., 110 of Fig. 1) and a proximal pressure sensor (e.g., 102 of Fig. 1). The procedure of Fig. 3A recovers the stored signal segment S22 and processes it to remove noise S24 (e.g., by low-pass filtering, smoothing, thresholding, or other suitable filtering process). At S26, the pressure signal segment is analyzed to generate a reliability metric indicating its accuracy. The latter may be done in various ways, for example, by identifying differences between a stored actual reading and a measured pressure or rate of change in pressure. In addition, or alternatively, the goodness of fit of the pressure profile to a stored model may provide a measure of accuracy (the curves being fitted in S28). The pressure reading may be compared to a profile. In S28, pressure profile data is translated into a respiration rate and pulse rate by fitting expected respiration and pulse curves to the stored data and the reliability metric and analyzing.

More sophisticated analysis may be done in S28 as well, for example, by fitting the measured data curves to curves that characterize identifiable conditions, such as dangerous conditions. For example, a leak may be indicated by a sharp drop in pressure at the distal location along with a gradual trend of ebbing pressure. The profile templates that characterize events may be determined via experiment or modeling or simply by judgment and stored in a memory of the controller. Other events that may be identified, for example, by comparing distal and proximal pressure readings, are kinks or flow restrictions in the access line or changes in the properties of fluid, for example such as may evidence peritoneal infection. The latter may be detected by identifying an excessive pressure drop in the access line during a drain operation, which may be caused by excessive viscosity in the spent dialysate.

In S30, events detected in the profile data, current pressure values, historical data, and reliability estimates are updated. Current data, for example, may be stored in a location representing current values, and historical data may be stored in memory locations representing historical values along with time and date values. For example, a memory location may hold a current estimate of patency of the access line. The event detection results may be represented as status flags and associated reliability estimates or other metrics such as a measure of goodness of fit to a characteristic curve or instantaneous value.

Referring to Fig. 3C, during a fill or drain cycle S42, the event recognition status and/or instantaneous values, such as those of pressure, are read by the controller from the controller memory S44 and compared to various threshold levels S46, S48, S50, and if the threshold test is met, an alarm indication may be generated S52 and the cycler may be placed in a safe mode corresponding to the detected event or condition. Otherwise, control may return to S42.

Archived data may be transferred to a data store for combination with data of multiple patients, for example via an internet connection, for analysis and comparison purposes. The conditions detected in S46, S48, S50 may include, for example, a reduction in the strength of vital signs (e.g., respiration, pulse) signal evidencing a line obstruction, loss of patency of the catheter or other problem; excessive pressure loss for an instantaneous flow rate, which may indicate a line obstruction, kink, or pinching of the line or other problem; excessive pressure of the peritoneum which may be compensated by reducing or stopping flow; and/or excessive drain flow pressure loss in the drain line due to high viscosity which may indicate an infection.

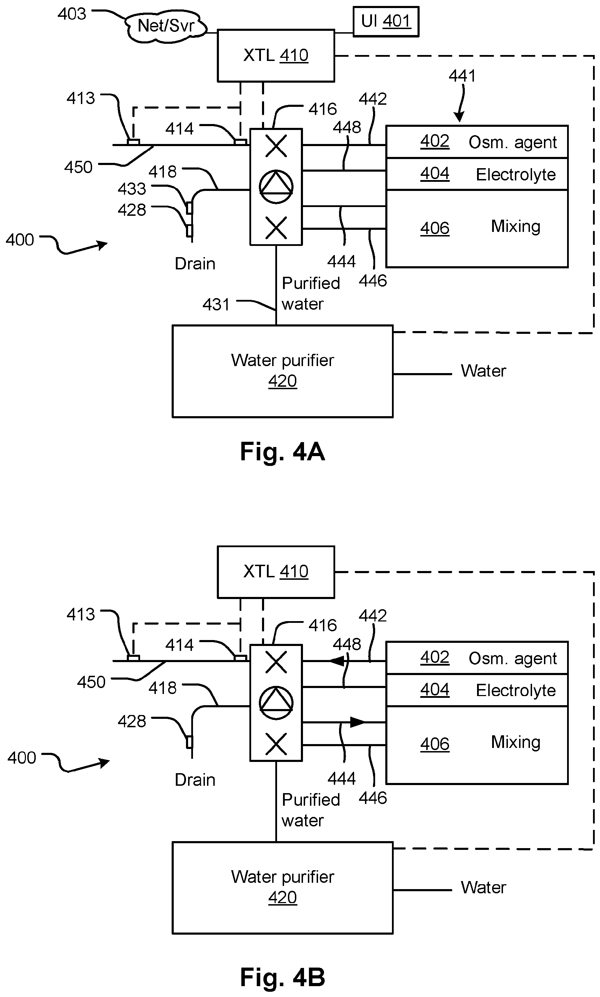

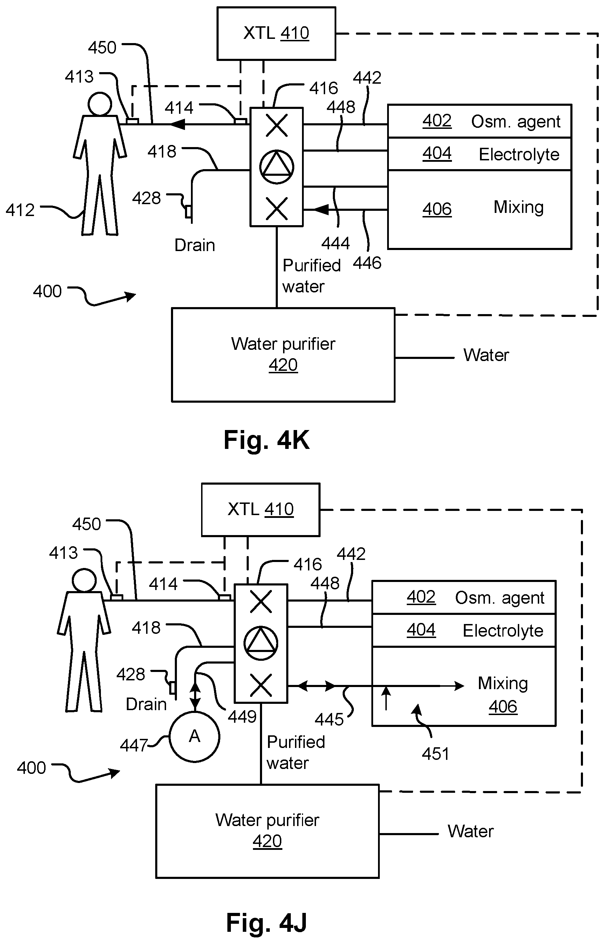

Fig. 4A shows a peritoneal dialysis fluid proportioner/cycler according to embodiments of the disclosed subject matter. The present Figs. 4A through 4H and 4K are generalizations of the various embodiments disclosed above for purposes of explaining the operational use thereof for preparing peritoneal dialysis fluid and for treating a patient using the structures described above. Referring now to Fig. 4A, a peritoneal dialysis fluid proportioner/cycler 400 may correspond to any of the foregoing embodiments described for generating dialysis fluid by mixing concentrates and water. For example, note Figs. 4A-D. Here, the peritoneal dialysis fluid proportioner/cycler 400 generates custom peritoneal dialysis fluid according to a prescription stored in a controller 410 (corresponding to controllers described above). The prescription may be entered in the controller via a user interface 401, via a remote terminal and/or server 403, or by other means such as a smart card or bar code reader (not shown). The controller applies control signals to a fluid conveyer and valve network 416 and a water purifier 420 and receives signals from distal pressure sensor 413 and proximal pressure sensor 414, respectively, on a fill/drain line 450 which may be in accord with foregoing embodiments.

The fluid circuit with pump and valve network 416 is a fluid circuit element with one or more sensors, actuators, and/or pumps which is effective to convey fluid between selected lines 442, 444, 446, 448, 450 and 418 responsively to control signals from the controller 410. Example embodiments are described herein, but many details are known from the prior art for making such a device so they are not elaborated here.

A multiple-container unit 441 includes a pre-filled, pre-sterilized osmotic agent concentrate container for osmotic agent concentrate 402 and another electrolyte concentrate container 404 for electrolyte concentrate. The multiple-container unit 441 also contains the mixing container 406 (which is empty) which is large enough to hold a sufficient volume of dialysis fluid for the completion of at least one fill cycle of an automated peritoneal dialysis treatment. The containers 402, 404, and 406 may be flexible bag-type containers that collapse when fluid is drawn from them and therefore, do not require any means to vent air into them when drained.

Osmotic agent concentrate container 402, electrolyte concentrate container 404, and mixing container 406 are all connected by respective lines 442, 448, 444, and 446 to the fluid circuit with pump and valve network 416. The fill/drain line (or multiple lines) 450 and a drain line 418 for spent fluid (and other fluids) with a conductivity sensor 428 may also be connected to the fluid circuit with pump and valve network 416. As shown in Fig. 4A, a further sensor 433 may be present on the drain line 418 to measure pressure and/or temperature of fluid in the drain line. The fluid circuit with pump and valve network 416 also has a purified water line 431 for receiving water. The water purifier 420 may be a purifier or any source of sterile and purified water including a pre-sterilized container of water or multiple containers. In a preferred configuration, water purifier 420 may be configured as described in WO2007/118235 (PCT/US2007/066251) and US20150005699. For example, the water purifier 420 may include the flow circuit components of Fig. 22A of WO2007/118235 including the water purification stages and conform generally to the mechanical packaging design shown in Fig. 24 of WO2007/118235.

It should be evident that 416 is a generalization of the peritoneal dialysis fluid proportioner/cycler 101 as well as elements of a fluid circuit such as fluid circuit 600 and connection platform 100. It should also be evident that 402 and 404 represent concentrate containers according to any of the disclosed embodiments. The mixing container 406 corresponds to any of the mixing container embodiments described above. Other elements will be evident from their description with the understanding that the figures represent generalizations thereof for purposes of describing the function. It should also be understood that the number and type of concentrates may differ from the present figure which is disclosed as an example, only. It should also be evident that the examples of concentrates discussed herein are glucose and electrolyte concentrates but they could be one or other multiples or other concentrates in other embodiments. Also, the osmotic agent concentrate or glucose concentrate is presumed here to include an electrolyte concentrate marker to permit the concentration of osmotic agent to be inferred from a measurement of conductivity of diluted agent with a priori knowledge (stored in a memory used by the controller) of the ratio of osmotic agent concentrate to electrolyte concentrate in the osmotic agent concentrate. See US20150005699. In alternative embodiments, the osmotic agent is not provided with an electrolyte marker and the peritoneal dialysis fluid proportioner/cycler 400 may rely on volumetric proportioning for the transfer of osmotic agent. Note also that the order of concentrate addition may be reversed, with electrolyte being added first.

Fig. 4B shows a preliminary stage of fluid preparation prior to peritoneal dialysis treatment according to an embodiment of the disclosed subject matter. The controller 410 reads a prescription and generates a command, responsive to a peritoneal dialysis treatment preparation initiation command, to flow osmotic agent concentrate from osmotic agent concentrate container 402 to the mixing container 406. The command is applied to the fluid circuit with pump and valve network 416 to connect the osmotic agent concentrate line 442 to the batch fill line 444 and also to convey the osmotic agent concentrate into the mixing container 406. This may be done by one or more valve actuators and one or more pumps that form the fluid circuit with pump and valve network 416. The fluid circuit with pump and valve network 416 may be configured to meter the quantity of osmotic agent concentrate precisely according to a predicted amount of dilution by electrolyte concentrate and water to produce the desired prescription fluid. The metering may be performed by a positive displacement pump internal to the fluid circuit with pump and valve network 416 or other means such as a measurement of the weight of the osmotic agent concentrate container 402 or the mixing container or a volumetric flow measurement device.

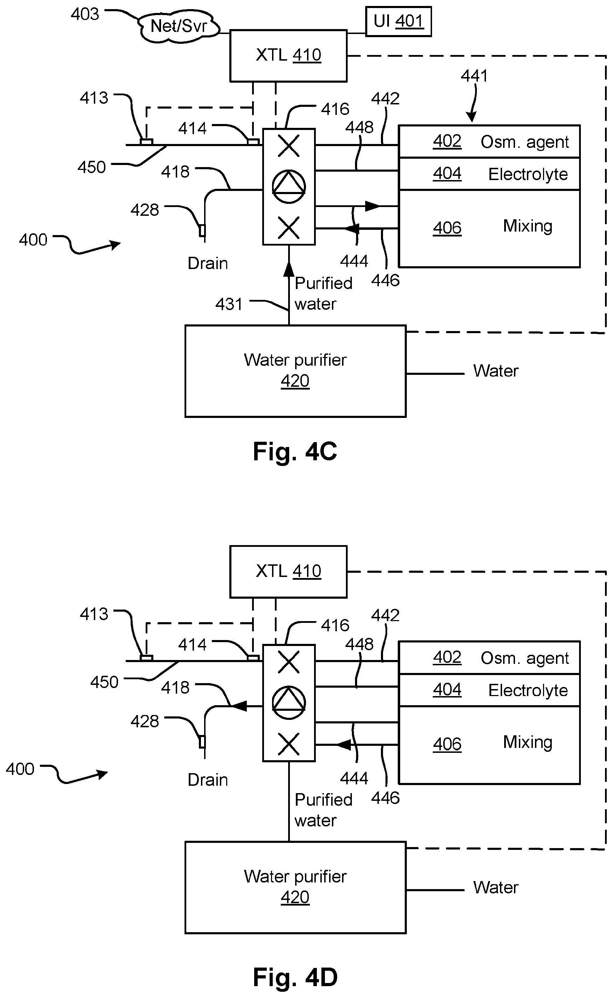

In an alternative embodiment, part of the water (less than the total used for dilution as discussed below with reference to Fig 4C) is added to the mixing container first, before the osmotic agent concentrate and electrolyte concentrates (if needed) are pumped into the mixing container.

Referring now to Fig. 4C, a dilution stage is performed using the peritoneal dialysis fluid proportioner/cycler 400. The controller 410, in response to the prescription, generates a command to flow purified water from the water purifier 420 to the mixing container 406. The command is applied to the fluid circuit with pump and valve network 416 to connect the purified water line 431 to the mixing container 406 to add a measured quantity of water to dilute the osmotic agent concentrate in the mixing container 406. The controller 410 may control the fluid circuit with pump and valve network 416 to ensure the correct amount of water is conveyed. Alternatively, the water purifier may incorporate a flow measurement device or metering pump or other suitable mechanism to convey the correct amount of water. The controller 410 may be connected to the water purifier 420 to effectuate the dilution result. The fluid circuit with pump and valve network 416 may also be configured to circulate diluted osmotic agent concentrate solution through lines 444 and 446 either simultaneously with the dilution or after the diluting water has been transferred to the mixing container 406 according to alternative embodiments. The circulation mixes the contents of the mixing container 406.

The relative amounts of water, osmotic agent concentrate, and electrolyte concentrate may be realized based on the ratiometric proportioning properties of the pump. Since a single pump tube is used to convey all the liquids into the mixing container, most sources of offset from predicted pumping rate (based on shaft rotations, for example) to actual pumping rate affect all the fluids roughly equally.

Referring now to Fig. 4D, the diluted osmotic agent concentrate solution in the mixing container 406 is tested to ensure that the correct concentration of osmotic agent is achieved. In an embodiment, the osmotic agent concentrate 402 has an amount of electrolyte concentrate to generate a conductivity signal using the conductivity sensor 428 when fluid from the mixing container 406 is conveyed by the fluid circuit with pump and valve network 416 to the drain line 418 past the conductivity sensor. The amount of electrolyte concentrate pre-mixed with the osmotic agent concentrate may be the lowest ratio of electrolyte concentrate to osmotic agent concentrate that a predetermined prescription may require. The fluid circuit with pump and valve network 416 may perform the function using one or more valve actuators and one or more pumps that form the fluid circuit with pump and valve network 416. The fluid circuit with pump and valve network 416 may be configured to meter the quantity of water precisely or the function may be provided by the water purifier 420. The controller 410 may add additional water or osmotic agent concentrate and test the conductivity again if the measured concentration of osmotic agent and/or electrolytes, if applicable, is incorrect. In addition to using a combined osmotic agent and electrolyte concentrate in osmotic agent concentrate container 402, in an alternative embodiment, the osmotic agent concentrate container 402 holds osmotic agent concentrate with no electrolytes and osmotic agent concentration is optionally measured directly by other means such as specific gravity (hydrometer), refractive index (refractometer), polarization, infrared absorption or other spectrographic technique.

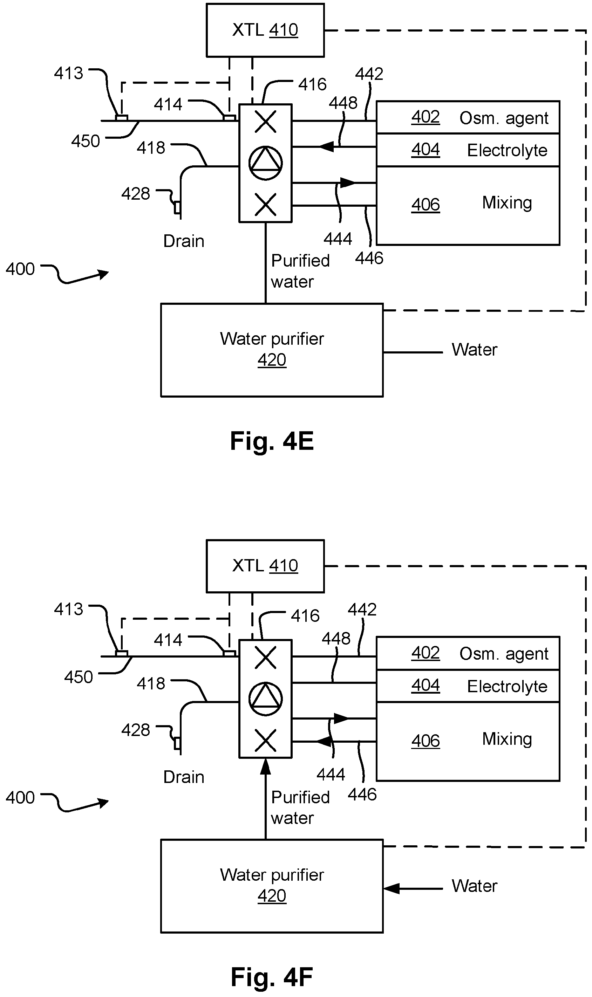

Fig. 4E shows an electrolyte concentrate addition stage of fluid preparation prior to peritoneal dialysis treatment according to an embodiment of the disclosed subject matter. The controller 410 reads a prescription and generates a command to flow electrolyte concentrate from container 404 to the mixing container 406. The command is applied to the fluid circuit with pump and valve network 416 to connect the electrolyte concentrate line 448 to the mixing container 406 fill line 444 and also to convey the electrolyte concentrate into the mixing container 406. This may be done by one or more valve actuators and one or more pumps that form the fluid circuit with pump and valve network 416. The fluid circuit with pump and valve network 416 may be configured to meter the quantity of electrolyte concentrate precisely according to a predicted amount of dilution by osmotic agent concentrate and water that has been previously determined to be in the mixing container 406, to achieve the prescription. The metering may be performed by a positive displacement pump internal to the fluid circuit with pump and valve network 416 or other means such as a measurement of the weight of the electrolyte concentrate container 404 or the mixing container 406 or a volumetric flow measurement device.

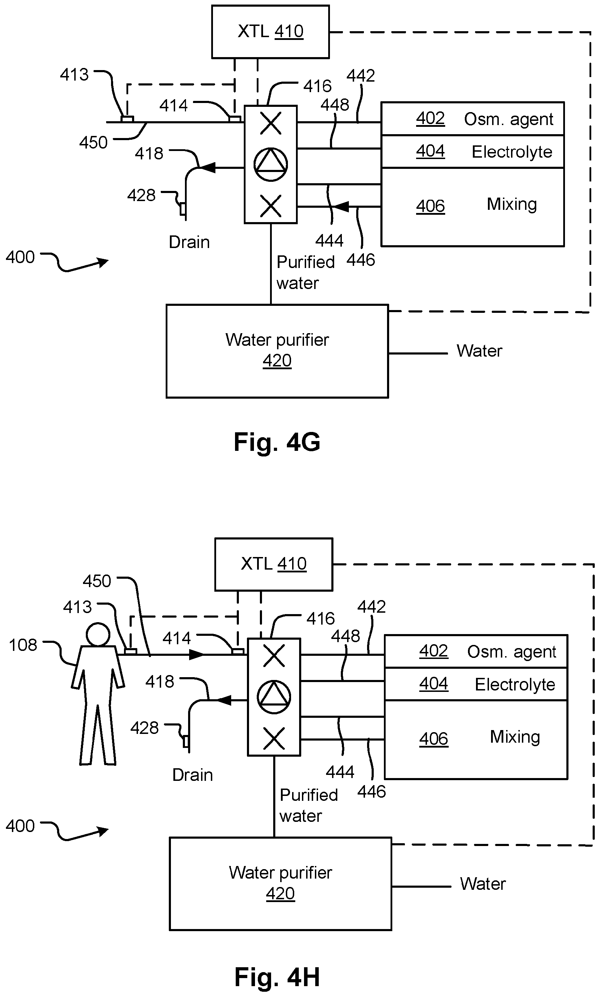

Referring now to Fig. 4F, the electrolyte concentrate may be mixed using the batch fill and drain lines 446 and 444 in a closed loop. If necessary, depending on how much dilution was performed during the osmotic agent concentrate dilution process, further dilution may be performed as described above. The final formulation may be achieved by the process illustrated in Fig. 4F. Then, as illustrated in Fig. 4G, the final electrolyte concentration of the mixture in mixing container 406 may be determined with a conductivity sensor 428 by flowing a sample therethrough.

Although gravimetric and tracer/conductance sensing were described as devices for ensuring proper proportioning and dilution rates for achieving target prescriptions, it should be clear that any embodiments of a peritoneal dialysis fluid proportioner/cycler disclosed herein may employ ratiometric proportioning as well, particularly where positive displacement pumping is employed. Ratiometric proportioning takes advantage of the volumetric repeatability and predictability of certain pumps. For example, a particular pump can deliver a highly repeatable volume of fluid for a given number of pumping cycles (pump rotations for a peristaltic pump or cycles for a diaphragm pump, for example). If all dialysis fluid components (water, osmotic agent concentrate, and electrolyte concentrate, for example) are delivered to the mixing container using the same pump, including, for example, the pumping tube segment of a peristaltic pump, then the volume ratios of the components will, after adjustment for potential flow path and/or viscosity differences as described below, be fully determined by the number of pump cycles used to convey each component.

Ratiometric proportioning may supplement or substitute for measurement of the fluid conductance or density or other measurements. To convert the number of pump cycles to actual displaced mass or volume, a calibration may be performed and/or flow path (including fluid properties) compensation parameters may be employed. The flow path compensation parameters may be respective to each particular fluid flow path and/or fluid type, or may be identical for all fluid paths and fluid types. To provide enhanced accuracy, one or more pump calibration and/or flow path compensation parameters may be generated through a calibration procedure. Typically, flow path compensation factors will be established and stored in non-volatile memory. Typically, one or more flow path calibration procedures will be performed when the peritoneal dialysis fluid proportioner/cycler is used by a patient. The calibration procedure may be performed after each new fluid set is installed, or before each batch preparation cycle, or even multiple times during the preparation of a single batch. A disposable fluid set may be installed every day. The calibration procedure may be done using water. The calibration may sequentially pump fluid through one or more of the stages provided in Table 1.

Table 1: Example stages for sequentially pumping fluid during calibration

| From | To |
| --- | --- |
| Water source | Drain |
| Mixing container | Drain |

(continued)

| From | To |
|---|---|
| Osmotic agent concentrate container | Drain |
| Electrolyte concentrate container | Drain |
| Patient access | Drain |
| Osmotic agent concentrate container | Mixing container |
| Electrolyte concentrate container | Mixing container |
| Water source | Mixing container |

In the calibration procedure, fluid is pumped between any or all of the paths identified above. A separate calibration coefficient may be generated for each of the paths. The calibration coefficient may be stored in a memory or non-volatile data store, for example, as a parameter representing the number of ml/ per pump rotation (or diaphragm pump cycle), or as a proportionality ratio relative to a particular reference flow path. The actual fluid quantity transported during the calibration step may be measured by any suitable device (flow sensor) including volume or mass measurement devices or direct flow rate measurement with integration, for example, using laser Doppler velocimetry, thermal transit time, magnetohydrodynamics, propeller hydrometer, positive displacement flow measurement, differential pressure through a resistance such as a venturi, nozzle, orifice plate, or other flow obstruction, variable area or rotameter, pitot or impact tube, vortex shedding frequency counting, ultrasonic, or other device. A particularly advantageous device for flow calibration is to measure the transit time of a fluid property perturbation between spaced fluid property sensors as described below. Any of the disclosed embodiments may employ a flow sensor in which at least the portion of which that carries fluid is disposable so that the flow rate (or total displaced fluid quantity) can be input to a controller while allowing the use of a disposable fluid circuit. Examples include an ultrasonic soft tube flowmeter made by Strain Measurement Devices (SMD) that non-invasively measure flow in soft tubing by means of slotted transducers in which a length of tubing can be inserted during fluid circuit installation. For cartridge embodiments, the PD cycler can employ a moving transducer stage that engages an exposed tube length of the cartridge after passive insertion of the cartridge.

The pumping system may also be sufficiently repeatable in a way that allows precise ratios to be established without calibration, depending on the predefined tolerances. If the manufacturing tolerances, including materials, are sufficiently controlled, a desired level of control over ratios may be achieved without in situ (point of care) calibration. A particularly sensitive component in terms of guaranteeing repeatability is the pumping tube segment of a peristaltic pump. In a first embodiment, the peristaltic pump tube segment is made from a material whose mechanical and material tolerances are controlled within predefined limits. The lengths of the tubing circuit elements and mechanical parameters are also controlled within respective predefined limits. A calibration may then be done outside the peritoneal dialysis treatment context, e.g., in the laboratory, to calculate precise values to convert pump cycles to fluid quantity transferred for a single lot of replaceable fluid circuits. The calibration may be done for multiple lots. The calibration may also be done for each fluid circuit. The calibration may also be done by the peritoneal dialysis fluid proportioner/cycler for each fluid circuit. The calibration may also be done for each batch of peritoneal dialysis fluid prepared by the fluid circuit.

Referring to Fig. 4H, subsequent to the preparation of the contents of the mixing container 406 as described above, the fluid circuit with pump and valve network 416 may be configured to drain the patient 108 depending on the patient's prior status. Spent dialysis fluid may be withdrawn by the fluid circuit with pump and valve network 416 and conveyed through the drain line 418. Then, the contents of the mixing container 406 may be conveyed as illustrated in Fig. 4K to the patient. Here the controller 410 has configured the fluid circuit with pump and valve network 416 to flow fluid to a patient 412.

Fig. 4K illustrates schematically a variation of the peritoneal dialysis fluid proportioner/cycler 400 of Fig. 4A with the addition of an accumulator 447 connected by an accumulator line 449 to allow a pump such as a peristaltic pump according to any of the disclosed embodiments, to provide mixing with a single mixing container line 445 connecting the mixing container 406. The controller 410 pumps fluid from the mixing container 406 to the accumulator 447 back and forth multiple times to mix the contents of the mixing container 406. This is in contrast to the disclosed embodiments in which two lines connect the mixing container 406 to the fluid circuit with pump and valve network 416. As indicated, use of a pump that has the ability to accumulate fluid, such as a diaphragm pump, may allow fluid to be pumped into and out of the mixing container 406 without a separate accumulator 447, by pumping fluid into the mixing container 406 from the diaphragm pump internal volume. Reference numeral 451 points to the arrows indicating spaced ingoing and outgoing flows to/from the mixing container that may be provided by the foregoing embodiments of devices for separating (at least partially) the ingoing and outgoing flows.

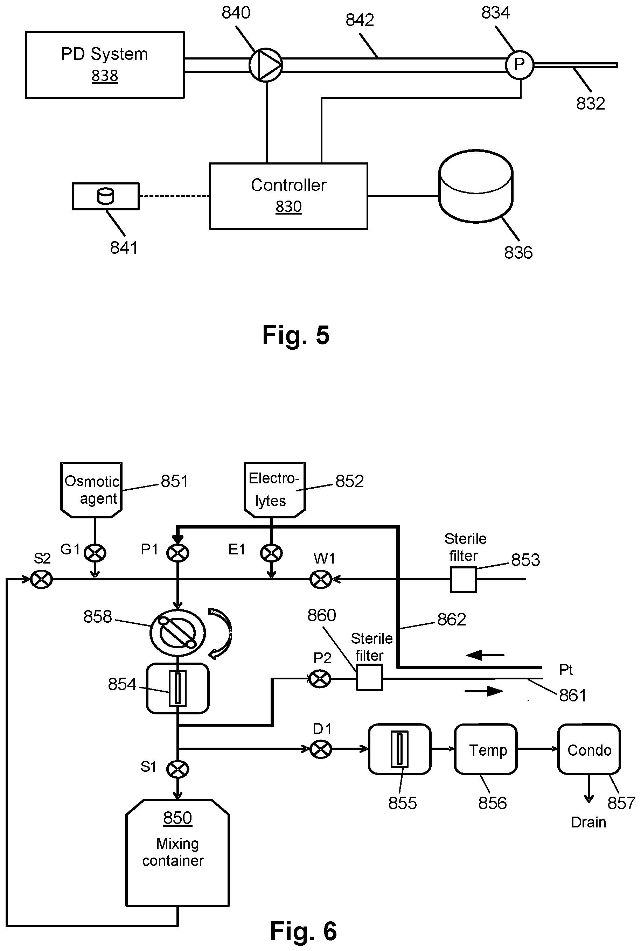

Fig. 5 illustrates a control system according to embodiments of the disclosed subject matter. A controller 830 may

8

receive sensor signals from any points in a PD system 838 including conductivity, temperature, and flow rate. The controller may apply actuator control signals to regulate the speed of pump 840 or an equivalent flow rate regulator such as a fixed rate pump with a variable recirculation bypass line or variable inline resistance such as a flow regulator valve. Fluid provided from the PD system 838 is transferred at a regulated rate to a peritoneal line 842, which may include a single line used for outgoing and return fluids or a pair of lines, each used respectively for outgoing and return fluids from patient connection line 832. A pressure sensor 834 generates signals indicating the pressure at a distal point in an outgoing peritoneal line or a peritoneal line that transfers fluids in both directions. An additional pressure sensor may be used for outgoing and return lines, respectively. A data store 836 may store one or more treatment profiles specific to a disposable unit that includes a fluid circuit (which may vary according to characteristics of the fluid circuit), specific to a particular patient or class of patients, or another requirement.

Pressure profile data stored on the data store 836 may be obtained from a data store 841 attached to the disposable unit or may be downloaded from a server based on identifying information on such a data store 841. Alternatively, pressure profile data may be stored on the data store 836 periodically and specific data to be used for a treatment may be selected from a user interface 401 of the controller during treatment (for example, data for a particular patient identified through the user interface and whose profile data is obtained from a repository of patient-specific treatment data). The pressure profile data may include a single pressure value representing a maximum pressure at the point of the pressure sensor 834 indicating a maximum pressure and serving as a limit on the pumping rate by pump 840 as controlled by the controller 830 as described according to any of the foregoing embodiments. The pressure profile data may include multiple pressure values representing respective phases of a peritoneal dialysis fill cycle. For example, the pressure values may correlate volume and pressure or number of pump rotations and pressure, thus defining a profile. For example, the rate may be ramped progressively up toward a maximum and then slowed gradually to balance the desires of speedy throughput and patient comfort.

Fig. 6 shows a fluid path and actuator layout according to embodiments of the disclosed subject matter. The present embodiment shows variations on the embodiments described above. For example, separate fill 861 and drain 862 lines are connected to the patient (by a single lumen or dual lumen peritoneal catheter). A sterile filter 860 is provided in the fill line. Another sterile filter 853 is provided on the purified water source line. One or more flow sensors 854 and 855 may be provided, for example, as shown, which may be used for error condition detection or for implementing a calibration procedure to derive the conversion of pump cycles to net displaced mass or volume respective of each flow path. A temperature sensor 856 and a conductivity sensor 857 may be provided on the drain line, downstream of actuator D1, as shown. The temperature sensor 856 and conductivity sensor 857 may be used to sample and test the content of mixing container 850, before the fluid from mixing container 850 is pumped into the patient, to verify the correct therapeutic concentration of the fluid. Similarly, the temperature sensor 856 and the conductivity sensor 857 can measure conductivity or resistivity of fluid that is withdrawn from the patient, and to thereby assess the quantity of ion exchange that took place in the patient's peritoneal cavity during a dwell period. Respective valves G1, P1, P2, S1, S2, W1, and D1 control the flow of fluids in the circuit. A pump 858 moves fluid in the circuit, which includes a mixing container 850, an osmotic agent container 851, and electrolyte container 852. The following table (Table 2) shows an embodiment of an operational procedure for the embodiments covered by Fig. 6. Any of these features may be combined in any of the foregoing embodiments to form additional embodiments. For example, the one or more flow sensors may be provided in the embodiment of Fig. 4A. The method embodiments may be modified to add a calibration procedure.

Table 2: Operational Procedure

| Mode Description | Pump Operation | Valve State | | | | | | | |
|---|---|---|---|---|---|---|---|---|---|
| | | G1 | E1 | W1 | S1 | S2 | P1 | P2 | D1 |
| 1. Prime Osmotic agent | Do until A pump cycles | O | X | X | X | X | X | X | O |
| 2. Prime Electrolyte | Do until A pump cycles | X | O | X | X | X | X | X | O |
| 3. Prime Water to Drain (flush concentrate) | Do until B pump cycles | X | X | O | X | X | X | X | O |
| 4. Prime Water to SAK | Do until C pump cycles | X | X | O | O | X | X | X | X |
| 5. Prime Mixing Circuit | Do until D pump cycles | X | X | X | O | O | X | X | X |
| 6. Prime SAK to Drain (measure flow rate) | Do until E pump cycles | X | X | X | X | O | X | X | O |
| 7. Prime Patient Line (V1) | Do until F pump cycles | X | X | X | X | O | X | O | X |

(continued)

| Mode Description | Pump Operation | Valve State | | | | | | | |
|---|---|---|---|---|---|---|---|---|---|
| | | G1 | E1 | W1 | S1 | S2 | P1 | P2 | D1 |
| 8. Prime Patient Line (V2) | Do until G pump cycles | X | X | X | X | X | O | X | O |
| 9. Add Osmotic agent to SAK | Do until H (calc) pump cycles | O | X | X | O | X | X | X | X |
| 10. Add Electrolyte to SAK | Do until I (calc) pump cycles | X | O | X | O | X | X | X | X |
| 11. Add Water to SAK | Do until J (calc) pump cycles | X | X | O | O | X | X | X | X |
| 12. Mix | Do until K (calc) pump cycles | X | X | X | O | O | X | X | X |
| 13. Test Sample (Temp/Condo/Flow) | Do until L pump cycles | X | X | X | X | O | X | X | O |
| 14. Rinse Fluid Path w/Dialysate | Do until O pump cycles | X | X | X | X | O | X | X | O |
| 15. Drain Patient | Do until N (calc) pump cycles OR PRES > Fill Pres Limit | X | X | X | X | X | O | X | O |
| 16. Fill Patient | Do until M (calc) pump cycles OR PRES > Drain_Pres_Limit | X | X | X | X | O | X | O | X |
| 17. Patient Dwell | Do until TIME COUNT | - | - | - | - | - | X | X | - |
| 18. Empty batch container | Do until P (calc) pump cycles | X | X | X | X | O | X | X | O |

In the second column "Pump Operation", the letters A, B, C, etc., refer to predefined values. For example, a pump may rotate once for every 2 ml pumped so the values may correspond to an amount of fluid pumped. The columns labeled "Valve State" refer to the status of the valve as labeled in Fig. 6, with "X" referring to a closed condition and "O" referring to an open condition. The term "calc" in the "Pump Operation" column indicates that the number of pump cycles is adjusted according to a parameter obtained from calibration.

Any of the above systems may be modified so that an additional line is provided in the fluid circuit, and valved in the same way as the batch container but which leads to an auxiliary port. At the end of a cycler-assisted treatment cycle, a batch of fresh dialysate may be prepared and dispensed from this auxiliary port for use in continuous ambulatory peritoneal dialysis. In this system and method, the patient may end a cycler-assisted treatment, for example, a nocturnal treatment, with a filled peritoneum. After filling the peritoneum, an additional batch of dialysate may be prepared and pumped from the batch container to a secondary container through the auxiliary port. This may then be used for a second cycle of continuous ambulatory peritoneal dialysis (CAPD) after draining the spent dialysis with which the peritoneum was filled at the end of the cycler-assisted treatment phase. It should be readily apparent how an additional valve and connector on the treatment/fluid preparation device may be included in to allow fluid to be conveyed from the batch container as required for implementation. The controller of the treatment/fluid preparation device may be configured to perform this function automatically at a user's specified option which may be indicated through a user interface selection. Multiple batches for CAPD may also be dispensed in this manner. In addition, the batch container may be large enough for mixing enough dialysate for one or more CAPD treatment cycles on top of the last batch used for filling the peritoneum after completion of the phase of cycler-assisted peritoneal dialysis. In this way, the one or more CAPD batches may be prepared while the patient is still connected to the cycler and undergoing cycler-assisted therapy.

In any of the described embodiments, the osmotic agent may be, or include, glucose, L-camitine, glycerol, icodextrin, or any other suitable agents. Further, the components combined to make a peritoneal dialysis solution may vary in number and any of the embodiments described could be made from single concentrate components or any other number of concentrate components by straightforward modifications of the embodiments. For example, a buffer (e.g., acetate, bicarb, lactate) may be separate from an electrolyte which may be separate from an osmotic agent.

In any of the disclosed embodiments, pressure signals at proximal and distal ends of the peritoneal line may be generated while a no-flow, or low-flow, condition exists. This may be controlled to occur at a certain point in preparation for treatment, or during treatment, to generate indications of static hydraulic head in the line. For example, if a patient falls out of bed, and a sudden height difference between the proximal and distal ends arises, a pressure difference may be detected. The detection may trigger an alarm or other output and may instantiate a change in machine status for example a shutdown. Another inference from an out of bounds pressure difference during low or no flow is abnormal set up of the system. In embodiments, the conversion of pump cycles to total transferred flow may be governed by assumed system configuration which may include a certain range of height differences between the proximal and distal ends of the peritoneal line. The following table (Table 3) shows some possible behaviors.

Table 3: Possible Behaviors

| Machine status | Detected conditions | Response |
|---|---|---|
| Low or no flow (e.g., dwell) | DP outside range A | Generate alarm indicating misconfiguration. |
| Fill | DP outside range B | Generate alarm indicating misconfiguration |
| Fill | DP outside range C | Adjust flow rate and/or shut down flow. |
| Drain | DP outside range D | Generate alert message indicating possible infection. |
| Drain | DP outside range E | Generate alarm indicating misconfiguration |
| Drain | DP outside range F | Adjust flow rate and/or shut down flow. |
| Any time the line is filled with fluid | Pulse or respiration detected, or stronger than threshold G, at Proximal sensor | Indicate status of connection is ok. |
| Any time the line is filled with fluid | Pulse or respiration not detected or weaker than threshold G at Proximal sensor and is detected at distal sensor | Indicate connection is misconfigured or possibly misconfigured. |
| Dwell | Pulse or respiration detected, or stronger than threshold H, at Proximal sensor | Indicate status of connection is ok. |
| Dwell | Pulse or respiration detected, or weaker than threshold H, at distal sensor | Indicate connection is misconfigured or possibly misconfigured. |
| Any time line is filled with fluid | Pulse or respiration detected at distal sensor and not at proximal sensor | Indicate line is misconfigured or possibly misconfigured. |
| Fill | Proximal P high, distal P low | Indicate obstruction between |

In Table 3, ranges identified by letter may represent pressure profiles, that is pressure values (upper and lower limits or just upper or just lower limits) that change during a progressive process. For example, pressure range C may ramp up with the number of pump cycles. The range data may be stored in a memory of the controller and/or may be stored on a memory device of the replaceable tubing set and/or may be read from a remote server or derived by any other suitable system. The pressure range data may be respective to a particular tubing set model, treatment type, and/or patient, and selection may be automated or made manually through a user interface. The term misconfiguration can refer to kinks, obstructions, leaks, disconnections, or other types of line problems. In the table, anywhere alarm or other output is indicated as an action, this may include, or be in the alternative, instructing the user to take some action to verify the problem or a detailed explanation of what the action might be, for example, if a misconfiguration of the connection is indicated.

In any of the disclosed embodiments, the distal pressure sensor may be located within a peritoneal cycler machine or on the tubing set leading to the patient and close to the machine. The distal pressure sensor may be located near the patient and on the tubing set or within a peritoneal catheter. It may also be separated from the tubing set and positioned within the peritoneum. In such an embodiment, the pressure sensor lines may be attached to the tubing set. For example, metallized surface of the tubing or a co-extrusion (wire insulation and tubing being coextruded) may be attached to the tube at points therealong.

Pressure Regulation During PD Access Cycles

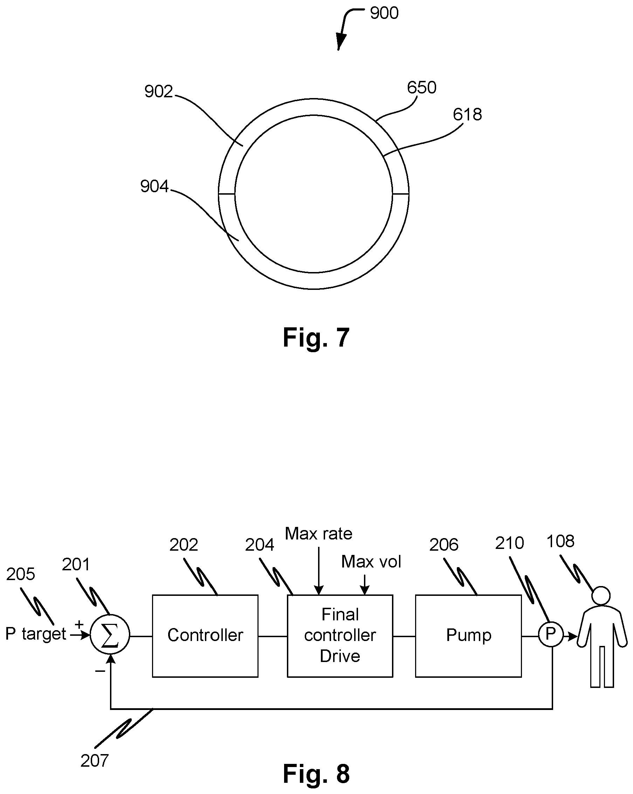

Embodiments provide closed-loop pressure control during the PD fill and/or drain cycles. Referring back to Fig. 4A, closed-loop pressure control is implemented by the controller 410 during a fill or drain cycle by obtaining pressure feedback from one or both of the distal and proximal pressure sensors 414 and 413 and controlling the pump rate of the pump in the fluid conveyer and circuit switch 416 accordingly. See Fig. 8, where a controller 202 generates a control signal based on a sum (by a summer 201) of a pressure target 205 and a negative input 207 from a pressure sensor 210. The controller output maintains the flow such that the target pressure 205 is maintained at the pressure sensor 210 by increasing or reducing pump 206 speed. A final controller and motor drive 204 imposes constraints on the controller signal to limit the rate or total volume such that if the controller runs the pump too fast, the pumping rate hits a ceiling and if a total volume of fluid is transferred, the pump is stopped. See below for more on this scheme. Note that the pressure sensor 210 may be located at various locations as identified below. These may include the pump inlet, the pump outlet, the proximal and distal ends of the access line or a combination of the proximal and distal end pressures such as a maximum of the two, a difference between the two and/or including a weighted average of the two. Pump inlet pressure may be used during drain and pump outlet pressure may be used during fill.

In one embodiment, during a drain cycle, based on the pressure feedback from one or both of the distal and proximal pressure sensors 414 and 413, the controller 410 controls the pump to drain to a constant pressure and then stop the draining when the pump rate hits some minimum value. Another condition is where the flow rate reaches some value higher than the minimum value for a predefined duration. Similarly, during a fill cycle, based on the pressure feedback from one or both of the distal and proximal pressure sensors 414 and 413, the controller 410 controls the pump to fill to a constant pressure and then stop the filling when a predefined volume of fluid is delivered. If the pump rate hits some minimum value it may be a result of an occlusion in the patient line.

During a fill or drain cycle, the controller 410 may operate a pump in one or both of two pumping modes: Volume Control Mode (VCM) and Pressure Controlled Rate Limiting (PCRL) mode. In the VCM mode, the pump is set to deliver a certain volume of fluid, and the controller 410 operates the pump at a certain rate (ml/min) until the target volume is reached. In the PCRL mode, the controller 410 sets at least two parameters for the pump operation: a target pressure and a maximum flow rate. The controller 410 operates the pump at the maximum flow rate to reach the target pressure, and then controls the pump to maintain the target pressure. If the pump does not, or cannot, reach the target pressure, the controller 410 continues to run the pump at the maximum flow rate. Either way, the controller 410 stops the pump when a target volume is reached.

In the PCRL mode, both pump parameters (the target pressure and the maximum flow rate) may be changed during a fill or drain cycle. For example, either one or both parameters may be changed at set times, volumes, or pressures. Further, either one or both parameters may be reset and limited in response to pressure or rate changes and/or other "deltas" determined with reference to "current" values. In embodiments, the maximum target pressure and flow rate (and/or the deltas) may be adjusted to reflect patient "comfort settings" as described in detail later herein.

In embodiments, for example, a change in the parameters may be triggered when the treatment approaches the end of a cycle (i.e., estimated time of end of cycle or as indicated by the lowering of flow in the pressure-controlled flow regime, or the net fluid transferred at the current point). In respective embodiments, the approach to end of cycle may be indicated based on a current volume of fluid displaced and/or based on a current pressure (detected by 413 and/or 414) falling below a threshold or by a pressure-regulated flow rate falling below a threshold. In response, the controller 410 may slow down the pump. As another example, a change in the parameters may be triggered when the patient shifts position and thereby causes a sudden free flow. In this case, the parameters may be adjusted to prevent a sudden large change in the pump rate, and the parameters may be gradually changed back to normal values over a period of time.

As another example, a change in the parameters may be triggered when a pattern of events is detected. Patterns may be stored for each patient with corresponding feedback from the patient. Patterns may be characterized based on a mathematical algorithm such as a fit to a basis function or functions such as a power series or Fourier series and associated to corresponding levels of comfort or discomfort, such as a five star scale. Over time the parameters defining the function (e.g., coefficients) may be correlated reliably with estimates of comfort to determine the best fill and/or drain patterns to employ in current and future cycles. Thus, a personalized set of parameters may be developed based on input feedback from the patient. The feedback may be provided based on a slider feedback that allows for selecting, for example, gentle and normal operation.

As another example, a change in the parameters may be triggered due to a detection of lumpiness of flow. For example, the pump may first be started with a fast flow. After a period, the pressure in the line proximate the peritoneal cavity drops a predefined magnitude in a predefined time interval or vacillates in a way that indicates the fluid is lumpy.

One embodiment provides pressure relief at the end of a drain cycle to mitigate negative pressure. This may be accomplished, for example, by reversing the pump, by stopping the pump and opening certain valves to relieve pressure, or by moving the distal POD 414 membrane to relieve pressure (i.e., operating the distal POD 414 as a diaphragm pump).

In embodiments, the controller 410 may detect and account for external effects when performing closed-loop pressure control. For example, the controller 410 may monitor an audio signal captured by a microphone proximate to the peritoneal cycler system 400, and identify relevant audio events such as coughs, sneezes, etc. Then, the controller 410 may filter such events out of the closed-loop pressure control algorithms. For example, when the audio signal indicates a cough, the controller 410 may temporarily ignore any changes in the pressure detected by pressure sensors 413 and/or 414.

In embodiments, the controller 410 may determine a viscosity of the fluid based on the pressure difference between pressure indicated by pressure sensors 413 and 414 and perform closed-loop pressure control by accounting for such viscosity.

In embodiments, the peritoneal cycler system 400 may provide a menu of user-selectable items (e.g., via the user interface 401) that allows a user to enter/select "comfort level" settings for fill and/or drain cycles. Such selectable items may include, for example, any of the pump rate and pressure settings/thresholds described above. The settings may allow for a user-selectable tradeoff between the access cycle time and the level of comfort caused by pump rate/pressure. For example, at the expense of lengthening the duration of an access cycle, a patient may dial down the pump rate or pressure. Alternatively, a patient may accept a less comfortable pump rate/pressure setting during an access cycle so that the access cycle may end more quickly and in a shorter time.

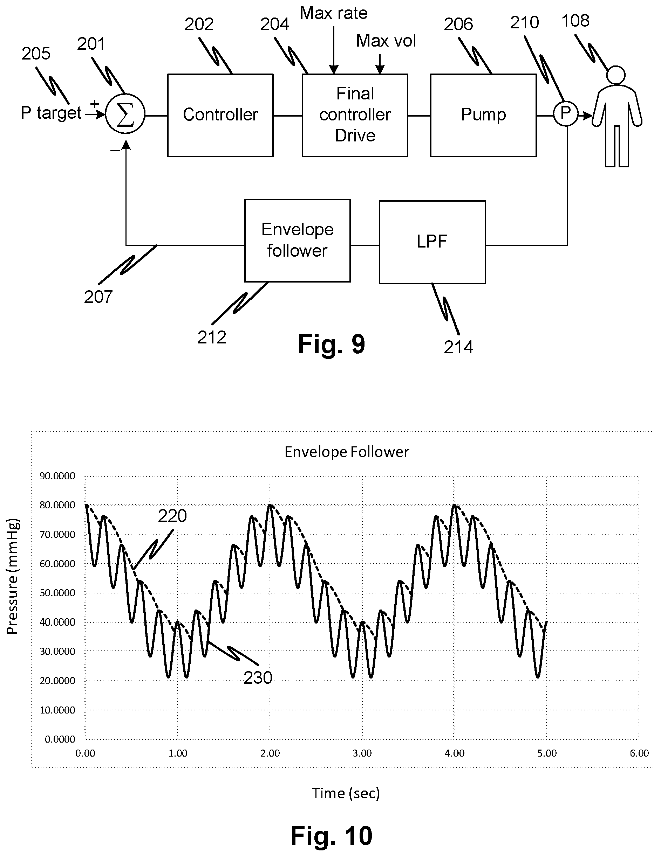

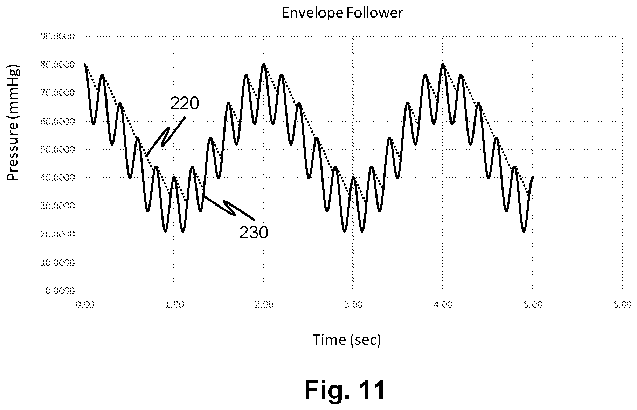

In embodiments, a pressure signal is applied as a negative feedback signal 207 to a summer. The negative feedback signal 207 may be filtered through an envelope follower 212 which has the effect of shifting the pump output pressure signal upwardly during fill cycles and downwardly during drain cycles, thereby decreasing a rate of pumping in the respective directions. Fig. 10 shows the profile of the envelope follower signal 220 relative to the input pressure signal 230 while it is unconditioned by a low pass filter. The high frequency variations are due to pressure pulses from the pump, for example roller strikes of a peristaltic pump, while the low frequency component is due to unpredictable variations in pressure which could be a result, for example, of the patient's movements. The regularity of the low-frequency component is figurative and not representative of a typical pressure variation. The follower signal 220 is governed in this case by an exponential decay function. Fig. 11 shows an envelope function governed by a linear decay function. The result of using the envelope follower signal is that the rate of pumping is decreased nearer to the maximum pressure experienced by the patient by increasing the negative feedback signal. During the drain cycle the signals would be inverted. The effect on fill is that the envelope follower signal biases the pumping rate to compensate toward a lower pumping rate. The signal from the pressure sensor may also be low-pass filtered by a low-pass filter 214 prior to being applied to the envelope follower 212. Alternatively, or in addition, the output of the envelope follower 212 may be applied to a low-pass filter (not shown) before being applied to the summer 201. It effectively follows the peaks of the pressure signal.

The PCLR system may be applied to variety of different types of pumps and although the embodiments contemplate peristaltic pumps, the method and system can be applied to other types of pumps. Also, the system may be used in any kind of system so while the above describes a mixing system that generates its own dialysis fluid, it could be used with prepackaged dialysis fluid as well as systems that make the fluid at the point of use.

Note that the pressure sensor used for closed-loop control may be a signal derived from a pressure near the patient (distal end of the access line), or a pressure sensor near the pump/cycler (proximal end of the access line). Alternatives also include a signal from measurements indicative of a value derived from both pressure sensors.

Note that in the embodiment of Fig. 8, the negative input 207 may be filtered by a low pass filter before being applied to the summer 201. In the embodiment of Fig. 9 the signal can be both low-pass filtered on input as well as low-pass filtered on output from the envelope follower.

Note that the control scheme of Figs. 8 and 9 applies to both fill and drain. Any of the embodiments may be applied to both cycles.

The pressure sensor 210 may be located at a variety of positions including at the patient, at the pump inlet during draining, and at the pump outlet during fill.

The scheme may be applied to a so-called push-back cycle where, during drain, fluid is pushed back to the peritoneum to attempt to differentiate between an empty patient and an occluded fluid line. As noted above, the control scheme naturally causes the pumping rate to slow down as the end of a drain cycle approaches. In embodiments a floor may be established such that if the flow rate drops to a predefined level before a targeted volume of fluid has been removed, the pump is stopped and an alarm output. This might happen due to an obstruction.

Note that a maximum rate may be present or not in any of the embodiments. There may be a maximum volume for both fill and drain cycles. For example, the maximum volume may be a patient fill volume according to a prescription. The drain maximum volume may be a double the patient fill volume or some other value. Note that if the drain maximum volume is exceeded it could indicate excess fluid in the peritoneum such as may be caused by ascites or some other condition. Upon reaching the maximum drain volume the system may output an alarm or other output to indicate the unexpected volume.

The target pressure may be set to a variety of different values in any of the embodiments. For example it may be set to a value that approximates the pressures associated with fill and/or drain measured at the patient transfer set during continuous ambulatory peritoneal dialysis (CAPD).

The feedback signal generated by the envelope follower may be used to drive a rate-limited pumping system where a parameter is used to control the maximum pressure change the envelope follower can track in a given time increment. The time increment may be a governable parameter. The time increment parameter is defined by a parameter that is modified automatically in response to the rate commanded by the controller. For a pump the time increment T may be equal to:

$$T = \cfrac{1}{\cfrac{pumping\ rate\ cmd\ \left[\frac{ml}{min}\right]}{\left\{\cfrac{dosing\ \left[\frac{ml}{rev}\right]}{60\ \left[\frac{sec}{min}\right]}\right\}} * roller\ hits/rev\ \left[\frac{hits}{rev}\right]}$$

The envelope follower may use an exponential decay function to define the signal envelope between maxima or minima. The envelope follower may use a linear decay function to define the signal envelope between the maxima and minima.

Measured Air During PD Drain

Generally, it is desirable to be able to convey predefined quantities of fluid into a patient for treatment purposes and to measure the amount of fluid withdrawn for purposes of measuring ultrafiltration and to avoid overfilling the peritoneal cavity. Accordingly, the controller 410 controls the total transferred volume of fresh dialysate to the patient as well as the total transferred volume of spent dialysate from the patient. One way to estimate such fluid volumes is based on the total volume displaced by a pump in the fluid conveyer and circuit switch 416. However, there may be air mixed with the spent fluid within the peritoneal cavity of the patient, for example, due to pneumoperitoneum (presence of abnormal/excessive gas in the peritoneal cavity). Therefore, the volume displaced by the pump includes both spent dialysate and air. As such, embodiments adjust the PD drained fluid volume estimates for cumulative measurement of air in fluid lines.

During a PD drain cycle, embodiments estimate the air removed from the patient by a pump, and subtract that estimate from the total volume displaced/removed (mix of fluid and air) to obtain the net volume of fluid removed from the patient. Referring back to Fig. 6A, the controller 410 detects the cumulative removed air volume based on readings from an air detector 650 (e.g. ultrasound type detector, optical detector, or any other air detector) disposed on the spent fluid drain line 618. Fig. 7 shows a cross-sectional view 900 of the spent fluid drain line 618 where the air detector 650 disposed thereon and surrounding the outer circumference of the spent fluid drain line 618. In embodiments where ultrasound air detectors are used, the ultrasound transmitter 902 transmits ultrasound waves through spent fluid drain line 618. The waves are propagated through fluid and/or air within the spent fluid drain line 618 and then received by the ultrasound receiver 904. Based on the phase shift between the transmitted and received waves, the controller 410 may determine the what fraction of the volume within the spent fluid drain line 618 is fluid and what fraction is air at any given time. The Controller 410 may do this based on a pre-populated look-up table that relates the phase shift with such proportions. By continuously making this determination and averaging over time, the controller 410 may determine the amount of air mixed with the fluid that is removed from the patient over a drain cycle.

In embodiments, the controller 410 may store an intraperitoneal pressure-vs-fill volume curve as a baseline for a given patient and compare a current curve to the baseline in order to detect reduced peritoneal volume due to adhesions or constipation. The controller may be configured to output a fill volume reduction relative to the baseline. Another baseline may be a fill pressure which may rise as a result of restricted flow due to adhesions in the peritoneum. The controller 410 may also be configured to detect an incorrect set up for initial drain by detecting a rapid decrease in peritoneal pressure indicating that a patient is already drained of dialysate (i.e., the patient is initially "dry"). The controller 410 may halt an initial drain and generate an error message indicating that the patient is "dry."

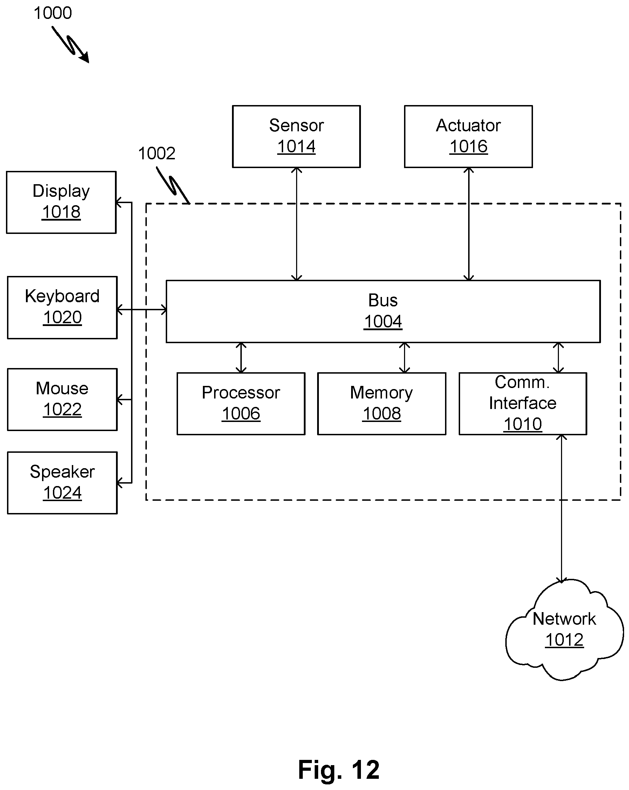

Fig. 12 shows a block diagram of an example computer system according to embodiments of the disclosed subject matter. In various embodiments, all or parts of system 1000 may be included in a medical treatment device/system such as a renal replacement therapy system. In these embodiments, all or parts of system 1000 may provide the functionality of a controller of the medical treatment device/systems. In some embodiments, all or parts of system 1000 may be implemented as a distributed system, for example, as a cloud-based system.

System 1000 includes a computer 1002 such as a personal computer or workstation or other such computing system that includes a processor 1006. However, alternative embodiments may implement more than one processor and/or one or more microprocessors, microcontroller devices, or control logic including integrated circuits such as ASIC.

Computer 1002 further includes a bus 1004 that provides communication functionality among various modules of computer 1002. For example, bus 1004 may allow for communicating information/data between processor 1006 and a memory 1008 of computer 1002 so that processor 1006 may retrieve stored data from memory 1008 and/or execute instructions stored on memory 1008. In one embodiment, such instructions may be compiled from source code/objects provided in accordance with a programming language such as Java, *C++, C#,* .net, Visual Basic™ language, LabVIEW, or another structured or object-oriented programming language. In one embodiment, the instructions include software modules that, when executed by processor 1006, provide renal replacement therapy functionality according to any of the embodiments disclosed herein.

Memory 1008 may include any volatile or non-volatile computer-readable memory that can be read by computer 1002. For example, memory 1008 may include a non-transitory computer-readable medium such as ROM, PROM, EEPROM, RAM, flash memory, disk drive, etc. Memory 1008 may be a removable or non-removable medium.

Bus 1004 may further allow for communication between computer 1002 and a display 1018, a keyboard 1020, a mouse 1022, and a speaker 1024, each providing respective functionality in accordance with various embodiments disclosed herein, for example, for configuring a treatment for a patient and monitoring a patient during a treatment.

Computer 1002 may also implement a communication interface 1010 to communicate with a network 1012 to provide any functionality disclosed herein, for example, for alerting a healthcare professional and/or receiving instructions from a healthcare professional, reporting patient/device conditions in a distributed system for training a machine learning algorithm, logging data to a remote repository, etc. Communication interface 1010 may be any such interface known in the art to provide wireless and/or wired communication, such as a network card or a modem.

Bus 1004 may further allow for communication with a sensor 1014 and/or an actuator 1016, each providing respective functionality in accordance with various embodiments disclosed herein, for example, for measuring signals indicative of a patient /device condition and for controlling the operation of the device accordingly. For example, sensor 1014 may provide a signal indicative of a viscosity of a fluid in a fluid circuit in a renal replacement therapy device, and actuator 1016 may operate a pump that controls the flow of the fluid responsively to the signals of sensor 1014.

While the present invention has been described in conjunction with a number of embodiments, the invention is not to be limited to the description of the embodiments contained herein, but rather is defined by the claims appended hereto and their equivalents. It is further evident that many alternatives, modifications, and variations would be or are apparent to those of ordinary skill in the applicable arts. Accordingly, Applicant intends to embrace all such alternatives, modifications, equivalents, and variations that are within the spirit and scope of this invention.

According to first embodiments, the disclosed subject matter includes a peritoneal dialysis system with a cycler component including a pump. A flow path switching mechanism engages with the cycler component and adapted to define multiple flow paths interconnecting a patient access line, fluid component and water lines, and a drain line. There is a pressure sensor located at one or more locations, said locations including at a distal end of the patient access line, the proximal end of the patient line, and somewhere between the proximal and distal ends of the patient line. A controller is configured to control the pump and the flow path switching mechanism to perform a cycler-assisted peritoneal dialysis treatment, the controller applying a closed-loop control on the pump based on a pressure feedback signal from the pressure sensor indicating the fluid pressure in the patient access line.

In variations thereof, the first embodiments include ones in which the controller applies the closed-loop control on the pump during a fill cycle of the cycler-assisted peritoneal dialysis treatment.

In variations thereof, the first embodiments include ones in which based on the pressure feedback signal from the pressure sensor, the controller controls the pump to fill to a constant pressure and then stops the pump when the pump rate reaches a pre-determined minimum value or when the pump rate remains at a predetermined lower value that is higher than said predetermined minimum value for at least a predetermined interval of time.

In variations thereof, the first embodiments include ones in which the controller applies the closed-loop control on the pump during a drain cycle of the cycler-assisted peritoneal dialysis treatment.

In variations thereof, the first embodiments include ones in which based on the pressure feedback signal from the pressure sensor, the controller controls the pump to drain to a constant pressure and then stops the pump a defined volume of fluid has been removed or when the pump rate reaches a pre-determined minimum value or when the pump rate remains at a predetermined lower value that is higher than said predetermined minimum value for at least a predetermined interval of time.

In variations thereof, the first embodiments include ones in which the controller controls a rate of the pump based on a comfort level set by a user.

In variations thereof, the first embodiments include ones in which the controller suspends the closed-loop control of the pump for a period of time when an audio event is indicated in the proximity of the system.

According to embodiments, the disclosed subject matter includes a peritoneal dialysis system with a cycler component including a pump. A flow path switching mechanism is engaged with the cycler component and adapted to define multiple flow paths interconnecting a patient access line, fluid component and water lines, and a drain line. An air detector is attached on the drain line. A controller is configured to control the pump and the flow path switching mechanism to perform cycler-assisted peritoneal dialysis treatment, the controller calculating a volume displaced by the pump, calculating an air volume based on readings of the air detector (e.g., ultrasound), and calculating a volume of fluid transferred from the patient by subtracting the air volume from the volume displaced.

According to second embodiments, the disclosed subject matter includes a peritoneal dialysis system with a cycler component including a pump, a patient access line, fluid component and a drain line, and a pressure sensor on the patient access line. A controller is configured to control the pump and to perform cycler-assisted peritoneal dialysis treatment including delivering fluid from the fluid component through the patient access line, the controller performing closed-loop control of the pump responsively to a pressure feedback signal from the pressure sensor indicating the fluid pressure in the patient access line.

In variations thereof, the second embodiments include ones in which the controller applies the closed-loop control on the pump during a fill cycle of the cycler-assisted peritoneal dialysis treatment.

In variations thereof, the second embodiments include ones in which based on the pressure feedback signal from the pressure sensor, the controller controls the pump to fill to a constant pressure and then stops the pump when a defined volume of fluid has been delivered or the pump rate reaches a pre-determined minimum value.

In variations thereof, the second embodiments include ones in which the controller applies the closed-loop control on the pump during a drain cycle of the cycler-assisted peritoneal dialysis treatment.

In variations thereof, the second embodiments include ones in which based on the pressure feedback signal from the pressure sensor, the controller controls the pump to drain to a constant pressure and then stops the pump when the pump rate reaches a pre-determined minimum value or when the pump rate remains at a predetermined lower value that is higher than said predetermined minimum value for at least a predetermined interval of time.

In variations thereof, the second embodiments include ones in which the controller controls a rate of the pump based on a comfort level stored in a memory and established for a predefined user.

In variations thereof, the second embodiments include ones in which the controller suspends the closed-loop control of the pump for a period of time when an audio event is indicated in the proximity of the system.

According to third embodiments, the disclosed subject matter includes a peritoneal dialysis cycler with a pump connected to a patient access line. At least one pressure sensor is connected to the patient access line to measure pressure of fluid delivered into said patient access line or drawn from it. A controller is configured to control the pump and to perform cycler-assisted peritoneal dialysis treatment including delivering fluid from the fluid component through the patient access line, the controller performing closed-loop control of the pump responsively to one or more pressure feedback signals from said at least one pressure sensor.

In variations thereof, the third embodiments include ones in which the one or more pressure feedback signal is a single signal from a distal pressure sensor near the patient end of the patient access line.

In variations thereof, the third embodiments include ones in which the one or more pressure feedback signal is a single signal from a proximal pressure sensor near the cycler end of the patient access line.

In variations thereof, the third embodiments include ones in which the one or more pressure feedback signal is a from a proximal pressure sensor near the cycler end of the patient access line and a signal from a distal pressure sensor near the patient end of the patient access line.

In variations thereof, the third embodiments include ones in which the feedback signal is a sum or average of the signals from the proximal and distal pressure sensors.

In variations thereof, the third embodiments include ones in which the feedback signal is a difference between the signals from the proximal and distal pressure sensors.

In variations thereof, the third embodiments include ones in which the feedback signal is low-pass filtered.

In variations thereof, the third embodiments include ones in which the feedback signal is an envelope of a signal from the identified at least one pressure sensor.

In variations thereof, the third embodiments include ones in which the feedback signal is an envelope of a low-pass filtered signal from the identified at least one pressure sensor.

In variations thereof, the third embodiments include ones in which the feedback signal is a low-pass filtered version of the envelope signal.

In variations thereof, the third embodiments include ones in which the feedback signal is a low-pass filtered version of an envelope of a low-pass filtered signal from the identified at least one pressure sensor.

In variations thereof, the third embodiments include ones in which the controller's closed-loop control target is a predefined pressure during a drain cycle.

In variations thereof, the third embodiments include ones in which the controller's closed-loop control target is a predefined pressure during a fill cycle.

In variations thereof, the third embodiments include ones in which the controller's closed-loop control target is a predefined pressure during push-back cycle.

In variations thereof, the third embodiments include ones in which the controller's closed-loop control output is limited by a predefined maximum commanded pumping rate.

In variations thereof, the third embodiments include ones in which the controller's closed-loop control output is limited by a predefined maximum flow rate.

In variations thereof, the third embodiments include ones in which the controller's closed-loop control output is limited by a predefined maximum total volume transferred.

In variations thereof, the third embodiments include ones in which the control target pressure is equal to a predefined equivalent CAPD pressure during fill.

In variations thereof, the third embodiments include ones in which the control target pressure is equal to a predefined equivalent CAPD pressure during drain.

According to fourth embodiments, the disclosed subject matter includes a peritoneal dialysis cycler with a pump connected to a patient access line. At least one pressure sensor is connected to the patient access line to measure pressure of fluid delivered into said patient access line or drawn from it. A controller is configured to generate a rate command that is applied to control the pump and to perform cycler-assisted peritoneal dialysis treatment including delivering fluid from the fluid component through the patient access line, the controller performing closed-loop control of the pump responsively to one or more pressure feedback signals from said at least one pressure sensor.

In variations thereof, the fourth embodiments include ones in which the feedback signal is an output of an envelope follower.

In variations thereof, the fourth embodiments include ones in which the controller accepts an input indicating a selectable maximum signal amplitude change the envelope follower can track in a given time increment.

In variations thereof, the fourth embodiments include ones in which the time increment is selectable responsively to an input.

In variations thereof, the fourth embodiments include ones in which the time increment is automatically selected responsively to a function of the pumping rate commanded by the controller.

In variations thereof, the fourth embodiments include ones in which the time increment T is equal to, for a peristaltic pump,

$$T = \frac{1}{\dfrac{pumping\ rate\ cmd}{dosing(ml.rev)/60\ sec/min} * roller\ hits\ per\ rev}$$

In variations thereof, the fourth embodiments include ones in which the envelope follower is defined in part by an exponential decay function.

In variations thereof, the fourth embodiments include ones in which the envelope follower is defined in part by a linear decay function.

In any of the foregoing embodiments, methods and systems and devices may be implemented using well-known digital systems. It will be appreciated that the modules, processes, systems, and sections described and/or suggested herein can be implemented in hardware, hardware programmed by software, software instruction stored on a non-transitory computer readable medium or a combination of the above. For example, a method for controlling the disclosed systems can be implemented, for example, using a processor configured to execute a sequence of programmed instructions stored on a non-transitory computer readable medium. For example, the processor can include a personal computer or workstation or other such computing system that includes a processor, microprocessor, microcontroller device, or is comprised of control logic including integrated circuits such as, for example, an Application Specific Integrated Circuit (ASIC). The instructions can be compiled from source code instructions provided in accordance with a programming language such as Java, C++, C#.net or the like. The instructions can also comprise code and data objects provided in accordance with, for example, the Visual Basic™ language, LabVIEW, or another structured or object-oriented programming language. The sequence of programmed instructions and data associated therewith can be stored in a non-transitory computer-readable medium such as a computer memory or storage device which may be any suitable memory apparatus, such as, but not limited to read-only memory (ROM), programmable read-only memory (PROM), electrically erasable programmable read-only memory (EEPROM), random-access memory (RAM), flash memory, disk drive and the like.

As used herein and in the claims, the term cycler-assisted peritoneal dialysis describes transferring fluid to the peritoneum of a living host and transferring fluid from the peritoneum of the host after a period of time.

One general aspect includes a peritoneal dialysis system. The peritoneal dialysis system also includes a cycler component including a pump. The system also includes a flow path switching mechanism engaged with the cycler component and adapted to define multiple flow paths interconnecting a patient access line, at least one fluid line, and a drain line. The system also includes a pressure sensor located at one or more locations, said locations including the proximal end of the patient access line, the distal end of the patient access line, somewhere between the proximal and distal ends, or within the cycler itself. The system also includes a controller configured to control the pump and the flow path switching mechanism to perform cycler-assisted peritoneal dialysis treatment, the controller applying a closed-loop control on the pump based on a pressure feedback signal from the pressure sensor indicating the fluid pressure in the patient access line. Other embodiments of this aspect include corresponding computer systems, apparatus, and computer programs recorded on one or more computer storage devices, each configured to perform the actions of the methods.

Implementations may include one or more of the following features. The peritoneal dialysis system where the controller applies the closed-loop control on the pump during a fill cycle of the cycler-assisted peritoneal dialysis treatment. Based on the pressure feedback signal from the pressure sensor, the controller controls the pump to fill to a constant pressure and then stops the pump when a defined volume of fluid has been delivered or the pump rate reaches a pre-determined minimum value. The controller applies the closed-loop control on the pump during a drain cycle of the cycler-assisted peritoneal dialysis treatment. Based on the pressure feedback signal from the pressure sensor, the controller controls the pump to drain to a constant pressure and then stops the pump when the pump rate reaches a pre-determined minimum value or when the pump rate remains at a predetermined lower value that is higher than said predetermined minimum value for at least a predetermined interval of time. The controller controls a rate of the pump based on a comfort level set by a user. The pump is a peristaltic pump. Implementations of the described techniques may include hardware, a method or process, or computer software on a computer-accessible medium.

One general aspect includes a peritoneal dialysis system. The peritoneal dialysis system also includes a cycler component including a pump. The system also includes a flow path switching mechanism engaged with the cycler component and adapted to define multiple flow paths interconnecting a patient access line, at least one fluid line, and a drain line. The system also includes an air detector on the patient drain line. The system also includes a controller configured to control the pump and the flow path switching mechanism to perform cycler-assisted peritoneal dialysis treatment, the controller calculating a volume displaced by the pump, calculating an air volume based on readings of the air detector, and calculating a volume of fluid transferred from the patient by subtracting the air volume from the volume displaced.

One general aspect includes a peritoneal dialysis system. The peritoneal dialysis system also includes a cycler component including a pump. The system also includes a patient access line, fluid component and a drain line. The system also includes a pressure sensor on the patient access line. The system also includes a controller configured to control the pump and to perform cycler-assisted peritoneal dialysis treatment including delivering fluid from the fluid component through the patient access line, the controller performing closed-loop control of the pump responsively to a pressure feedback signal from the pressure sensor indicating the fluid pressure in the patient access line. Other embodiments of this aspect include corresponding computer systems, apparatus, and computer programs recorded on one or more computer storage devices, each configured to perform the actions of the methods.

Implementations may include one or more of the following features. The peritoneal dialysis system where the controller applies the closed-loop control on the pump during a fill cycle of the cycler-assisted peritoneal dialysis treatment. Based on the pressure feedback signal from the pressure sensor, the controller controls the pump to fill to a constant pressure and then stops the pump when a defined volume of fluid has been delivered or the pump rate reaches a pre-determined minimum value. The controller applies the closed-loop control on the pump during a drain cycle of the cycler-assisted peritoneal dialysis treatment. Based on the pressure feedback signal from the pressure sensor, the controller controls the pump to drain to a constant pressure and then stops the pump when the pump rate reaches a pre-determined minimum value or when the pump rate remains at a predetermined lower value that is higher than said predetermined minimum value for at least a predetermined interval of time. The controller controls a rate of the pump based on a comfort level stored in a memory and established for a predefined user. The pump is a peristaltic pump. Implementations of the described techniques may include hardware, a method or process, or computer software on a computer-accessible medium.

One general aspect includes a peritoneal dialysis cycler. The peritoneal dialysis cycler also includes a pump connected to a patient access line. The cycler also includes at least one pressure sensor connected to the patient access line to measure pressure of fluid delivered into said patient access line or drawn from it. The cycler also includes a controller configured to control the pump and to perform cycler-assisted peritoneal dialysis treatment including delivering fluid from the fluid component through the patient access line, the controller performing closed-loop control of the pump responsively to one or more pressure feedback signals from said at least one pressure sensor. Other embodiments of this aspect include corresponding computer systems, apparatus, and computer programs recorded on one or more computer storage devices, each configured to perform the actions of the methods.

Implementations may include one or more of the following features. The cycler where the one or more pressure feedback signal is a single signal from a distal pressure sensor near the patient end of the patient access line. The feedback signal is low-pass filtered. The feedback signal is an envelope of a signal from the identified at least one pressure sensor. The feedback signal is an envelope of a low-pass filtered signal from the identified at least one pressure sensor. The feedback signal is a low-pass filtered version of the envelope signal. The controller's closed-loop control target is a predefined pressure during a drain cycle. The controller's closed-loop control target is a predefined pressure during a fill cycle. The controller's closed-loop control target is a predefined pressure during a push-back cycle. The controller's closed-loop control output is limited by a predefined maximum commanded pumping rate. The controller's closed-loop control output is limited by a predefined maximum flow rate. The controller's closed-loop control output is limited by a predefined maximum total volume transferred. The control target pressure is equal to a predefined equivalent capd pressure during fill. The control target pressure is equal to a predefined equivalent capd pressure during drain. Implementations of the described techniques may include hardware, a method or process, or computer software on a computer-accessible medium.

One general aspect includes a peritoneal dialysis cycler. The peritoneal dialysis cycler also includes a pump connected to a patient access line. The cycler also includes at least one pressure sensor connected to the patient access line to measure pressure of fluid delivered into said patient access line or drawn from it. The cycler also includes a controller configured to generate a rate command that is applied to control the pump and to perform cycler-assisted peritoneal dialysis treatment including delivering fluid from the fluid component through the patient access line, the controller performing closed-loop control of the pump responsively to one or more pressure feedback signals from said at least one pressure sensor. Other embodiments of this aspect include corresponding computer systems, apparatus, and computer programs recorded on one or more computer storage devices, each configured to perform the actions of the methods.

Implementations may include one or more of the following features. The cycler of any -40, where the envelope follower is defined in at least part by an exponential decay function. The envelope follower follows the input signal on a rising curve and is bound to fall as an exponential when the input signal drops. The envelope follower is defined at least in part by a linear decay function. The envelope follower follows the input signal on a rising curve and is bound to fall as a linear function when the input signal drops. The cycler further may include a proportioning system that generates peritoneal dialysis fluid at a location of treatment and immediately prior to the treatment. Implementations of the described techniques may include hardware, a method or process, or computer software on a computer-accessible medium.

Furthermore, the modules, processes, systems, and sections can be implemented as a single processor or as a distributed processor. Further, it should be appreciated that the steps mentioned above may be performed on a single or distributed processor (single and/or multi-core). Also, the processes, modules, and sub-modules described in the various figures of and for embodiments above may be distributed across multiple computers or systems or may be co-located in a single processor or system. Exemplary structural embodiment alternatives suitable for implementing the modules, sections, systems, means, or processes described herein are provided below.

The modules, processors or systems described above can be implemented as a programmed general purpose computer, an electronic device programmed with microcode, a hard-wired analog logic circuit, software stored on a computer-readable medium or signal, an optical computing device, a networked system of electronic and/or optical devices, a special purpose computing device, an integrated circuit device, a semiconductor chip, and a software module or object stored on a computer-readable medium or signal, for example.

Embodiments of the method and system (or their sub-components or modules), may be implemented on a general-purpose computer, a special-purpose computer, a programmed microprocessor or microcontroller and peripheral integrated circuit element, an ASIC or other integrated circuit, a digital signal processor, a hardwired electronic or logic circuit such as a discrete element circuit, a programmed logic circuit such as a programmable logic device (PLD), programmable logic array (PLA), field-programmable gate array (FPGA), programmable array logic (PAL) device, or the like. **In** general, any process capable of implementing the functions or steps described herein can be used to implement embodiments of the method, system, or a computer program product (software program stored on a non-transitory computer readable medium).

Furthermore, embodiments of the disclosed method, system, and computer program product may be readily implemented, fully or partially, in software using, for example, object or object-oriented software development environments that provide portable source code that can be used on a variety of computer platforms. Alternatively, embodiments of the disclosed method, system, and computer program product can be implemented partially or fully in hardware using, for example, standard logic circuits or a very-large-scale integration (VLSI) design. Other hardware or software can be used to implement embodiments depending on the speed and/or efficiency requirements of the systems, the particular function, and/or particular software or hardware system, microprocessor, or microcomputer being utilized. Embodiments of the method, system, and computer program product can be implemented in hardware and/or software using any known or later developed systems or structures, devices and/or software by those of ordinary skill in the applicable art from the function description provided herein and with a general basic knowledge of control systems and/or computer programming arts.

It is, thus, apparent that there is provided, in accordance with the present disclosure, peritoneal dialysis devices and systems. Many alternatives, modifications, and variations are enabled by the present disclosure. Features of the disclosed embodiments can be combined, rearranged, omitted, etc. Furthermore, certain features may sometimes be used to advantage without a corresponding use of other features.

**Claims**

**1.** A peritoneal dialysis cycler (101, 400), comprising:

a pump (206, 840, 858) connected to a patient access line (112, 450, 832);

at least one pressure sensor (102, 110, 413, 414, 834) connected to the patient access line (112, 450, 832) to measure pressure of fluid delivered into said patient access line (112, 450, 832) or drawn from it; and

a controller (116, 202, 410, 830) configured to control the pump (206, 840, 858) and to perform cycler-assisted peritoneal dialysis treatment including delivering fluid from a fluid component (106, 441, 850) through the patient access line (112, 450, 832), the controller (116, 202, 410, 830) performing closed-loop control of the pump (206, 840, 858) responsively to one or more pressure feedback signals from said at least one pressure sensor (102, 110, 413, 414, 834);

**characterized in that**

the feedback signal is an envelope of a signal from the at least one pressure sensor (102, 110, 413, 414, 834).

2. The cycler (101, 400) of claim 1, wherein the one or more pressure feedback signal is a single signal from a distal pressure sensor (110, 413, 834) near the patient end of the patient access line (112, 450, 832).

3. The cycler (101, 400) of claim 1, wherein the one or more pressure feedback signal is a single signal from a proximal pressure sensor (102, 414) near the cycler end of the patient access line (112, 450, 832).

4. The cycler (101, 400) of claim 1, wherein the one or more pressure feedback signal is from a proximal pressure sensor (102, 414) near the cycler end of the patient access line (112, 450, 832) and a signal from a distal pressure sensor (110, 413, 834) near the patient end of the patient access line (112, 450, 832).

5. The cycler (101, 400) of claim 4, wherein the feedback signal is derived using a sum or average of the signals from the proximal and distal pressure sensors (102, 110, 413, 414, 834).

6. The cycler (101, 400) of claim 4, wherein the feedback signal is derived using a difference between the signals from the proximal and distal pressure sensors (102, 110, 413, 414, 834).

7. The cycler (101, 400) of claim 1, wherein the feedback signal is an envelope of a low-pass filtered signal from the at least one pressure sensor (102, 110, 413, 414, 834).

8. The cycler (101, 400) of claim 1, wherein the feedback signal is a low-pass filtered version of the envelope signal.

9. The cycler (101, 400) of claim 1, wherein the feedback signal is a low-pass filtered version of an envelope of a low-pass filtered signal from the at least one pressure sensor (102, 110, 413, 414, 834).

10. The cycler (101, 400) of claim 1, wherein the controller's closed-loop control target is a predefined pressure during a drain cycle.

11. The cycler (101, 400) of claim 1, wherein the controller's closed-loop control target is a predefined pressure during a fill cycle.

12. The cycler (101, 400) of claim 1, wherein the controller's closed-loop control target is a predefined pressure during a push-back cycle.

13. The cycler (101, 400) of claim 1, wherein the controller's closed-loop control output is limited by a predefined maximum commanded pumping rate, by a predefined maximum flow rate or by a predefined maximum total volume transferred.

14. The cycler (101, 400) of claim 1, wherein the controller (410) monitors an audio signal captured by a microphone proximate to the peritoneal dialysis cycler (400) to identify relevant audio events and temporarily ignores any changes in the pressure detected by the at least one pressure sensor (413, 414) when the audio signal indicates a relevant audio event.

**Patentansprüche**

1. Peritonealdialyse-Zyklusgerät (101, 400), umfassend:

eine Pumpe (206, 840, 858), verbunden mit einer Patienten-Anschlussleitung (112, 450, 832);

mindestens einen Drucksensor (102, 110, 413, 414, 834), verbunden mit der Patienten-Anschlussleitung (112, 450, 832), um Druck von Fluid zu messen, welches in die genannte Patienten-Anschlussleitung (112, 450, 832) abgegeben oder aus ihr entnommen wird; und

eine Reglereinheit (116, 202, 410, 830), dazu ausgelegt, die Pumpe (206, 840, 858) zu regeln und Zyklusgerät-gestützte Peritonealdialysebehandlung durchzuführen, einschließlich Abgeben von Fluid von einer Fluidkomponente (106, 441, 850) durch die Patienten-Anschlussleitung (112, 450, 832), wobei die Reglereinheit (116, 202, 410, 830) Regelung der Pumpe (206, 840, 858) durchführt als Reaktion auf ein oder mehr Druck-Rückkopplungssignale von dem genannten mindestens einen Drucksensor (102, 110, 413, 414, 834); **dadurch gekennzeichnet, dass**

das Rückkopplungssignal eine Hülle eines Signals von dem mindestens einen Drucksensor (102, 110, 413, 414, 834) ist.

2. Zyklusgerät (101, 400) nach Anspruch 1, wobei das eine oder mehr Druck-Rückkopplungssignal ein einzelnes Signal von einem distalen Drucksensor (110, 413, 834) nahe dem Patienten-Ende der Patienten-Anschlussleitung (112, 450, 832) ist.

3. Zyklusgerät (101, 400) nach Anspruch 1, wobei das eine oder mehr Druck-Rückkopplungssignal ein einzelnes Signal von einem proximalen Drucksensor (102, 414) nahe dem Zyklusgerät-Ende der Patienten-Anschlussleitung (112, 450, 832) ist.

4. Zyklusgerät (101, 400) nach Anspruch 1, wobei das eine oder mehr Druck-Rückkopplungssignal von einem proximalen Drucksensor (102, 414) nahe dem Zyklusgerät-Ende der Patienten-Anschlussleitung (112, 450, 832) und ein Signal von einem distalen Drucksensor (110, 413, 834) nahe dem Patienten-Ende der Patienten-Anschlussleitung (112, 450, 832) ist.

5. Zyklusgerät (101, 400) nach Anspruch 4, wobei das Rückkopplungssignal unter Verwendung einer Summe oder eines Durchschnitts der Signale von den proximalen und distalen Drucksensoren (102, 110, 413, 414, 834) abgeleitet ist.

6. Zyklusgerät (101, 400) nach Anspruch 4, wobei das Rückkopplungssignal unter Verwendung einer Differenz zwischen den Signalen von den proximalen und distalen Drucksensoren (102, 110, 413, 414, 834) abgeleitet ist.

7. Zyklusgerät (101, 400) nach Anspruch 1, wobei das Rückkopplungssignal eine Hülle eines tiefpassgefilterten Signals von dem mindestens einen Drucksensor (102, 110, 413, 414, 834) ist.

8. Zyklusgerät (101, 400) nach Anspruch 1, wobei das Rückkopplungssignal eine tiefpassgefilterte Version des Hüllkurvensignals ist.

9. Zyklusgerät (101, 400) nach Anspruch 1, wobei das Rückkopplungssignal eine tiefpassgefilterte Version einer Hülle eines tiefpassgefilterten Signals von dem mindestens einen Drucksensor (102, 110, 413, 414, 834) ist.

10. Zyklusgerät (101, 400) nach Anspruch 1, wobei das Regelungsziel der Reglereinheit ein vordefinierter Druck während eines Entleerungszyklus ist.

11. Zyklusgerät (101, 400) nach Anspruch 1, wobei das Regelungsziel der Reglereinheit ein vordefinierter Druck während eines Füllzyklus ist.

12. Zyklusgerät (101, 400) nach Anspruch 1, wobei das Regelungsziel der Reglereinheit ein vordefinierter Druck während eines Zurückdrängenzyklus ist.

13. Zyklusgerät (101, 400) nach Anspruch 1, wobei die Regelungsleistung der Reglereinheit begrenzt ist durch eine vordefinierte maximale Soll-Pumprate, durch eine vordefinierte maximale Flussrate oder durch ein vordefiniertes maximales transportiertes Gesamtvolumen.

14. Zyklusgerät (101, 400) nach Anspruch 1, wobei die Reglereinheit (410) ein von einem Mikrofon nahe dem Peritonealdialyse-Zyklusgerät (400) erfasstes Audiosignal überwacht, um relevante Audioereignisse zu identifizieren, und vorübergehend jegliche Änderungen in dem von dem mindestens einen Drucksensor (413, 414) ermittelten Druck ignoriert, wenn das Audiosignal ein relevantes Audioereignis anzeigt.

## EP 4 061 447 B1

**Revendications**

1. Cycleur de dialyse péritonéale (101, 400), comprenant :

   une pompe (206, 840, 858) reliée à une ligne d'accès au patient (112, 450, 832) ;
   au moins un capteur de pression (102, 110, 413, 414, 834) connecté à la ligne d'accès au patient (112, 450, 832) pour mesurer la pression du fluide délivré dans ladite ligne d'accès au patient (112, 450, 832) ou aspiré de celle-ci ; et
   un contrôleur (116, 202, 410, 830) configuré pour commander la pompe (206, 840, 858) et pour effectuer un traitement de dialyse péritonéale assistée par cycleur, comprenant l'administration de fluide à partir d'un composant de fluide (106, 441, 850) à travers la ligne d'accès au patient (112, 450, 832), le contrôleur (116, 202, 410, 830) effectuant une commande en boucle fermée de la pompe (206, 840, 858) en réponse à un ou plusieurs signaux de rétroaction de pression provenant dudit au moins un capteur de pression (102, 110, 413, 414, 834) ; **caractérisé en ce que**
   le signal de rétroaction est une enveloppe d'un signal provenant de l'au moins un capteur de pression (102, 110, 413, 414, 834).

2. Cycleur (101, 400) selon la revendication 1, dans lequel les un ou plusieurs signaux de rétroaction de pression sont un signal unique provenant d'un capteur de pression distal (110, 413, 834) situé près de l'extrémité côté patient de la ligne d'accès au patient (112, 450, 832).

3. Cycleur (101, 400) selon la revendication 1, dans lequel les un ou plusieurs signaux de rétroaction de pression sont un signal unique provenant d'un capteur de pression proximal (102, 414) situé près de l'extrémité du cycleur de la ligne d'accès au patient (112, 450, 832).

4. Cycleur (101, 400) selon la revendication 1, dans lequel les un ou plusieurs signaux de rétroaction de pression proviennent d'un capteur de pression proximal (102, 414) situé près de l'extrémité du cycleur de la ligne d'accès au patient (112, 450, 832) et d'un capteur de pression distal (110, 413, 834) situé près de l'extrémité patient de la ligne d'accès au patient (112, 450, 832).

5. Cycleur (101, 400) selon la revendication 4, dans lequel le signal de rétroaction est dérivé à l'aide d'une somme ou d'une moyenne des signaux provenant des capteurs de pression proximal et distal (102, 110, 413, 414, 834).

6. Cycleur (101, 400) selon la revendication 4, dans lequel le signal de rétroaction est dérivé à l'aide d'une différence entre les signaux provenant des capteurs de pression proximal et distal (102, 110, 413, 414, 834).

7. Cycleur (101, 400) selon la revendication 1, dans lequel le signal de rétroaction est une enveloppe d'un signal filtré par un filtre passe-bas provenant d'au moins un capteur de pression (102, 110, 413, 414, 834).

8. Cycleur (101, 400) selon la revendication 1, dans lequel le signal de rétroaction est une version filtrée par un filtre passe-bas du signal d'enveloppe.

9. Cycleur (101, 400) selon la revendication 1, dans lequel le signal de rétroaction est une version filtrée par un filtre passe-bas d'une enveloppe d'un signal filtré par un filtre passe-bas provenant de l'au moins un capteur de pression (102, 110, 413, 414, 834).

10. Cycleur (101, 400) selon la revendication 1, dans lequel la cible de commande en boucle fermée du contrôleur est une pression prédéfinie pendant un cycle de vidange.

11. Cycleur (101, 400) selon la revendication 1, dans lequel la cible de commande en boucle fermée du contrôleur est une pression prédéfinie pendant un cycle de remplissage.

12. Cycleur (101, 400) selon la revendication 1, dans lequel la cible de commande en boucle fermée du contrôleur est une pression prédéfinie pendant un cycle de refoulement.

13. Cycleur (101, 400) selon la revendication 1, dans lequel la sortie de commande en boucle fermée du contrôleur est limitée par un débit de pompage maximal prédéfini, par un débit maximal prédéfini ou par un volume total transféré maximal prédéfini.

14. Cycleur (101, 400) selon la revendication 1, dans lequel le contrôleur (410) surveille un signal audio capturé par un microphone à proximité du cycleur de dialyse péritonéale (400) afin d'identifier des événements audio pertinents, et ignore temporairement toute variation de la pression détectée par les un ou plusieurs capteurs de pression (413, 414) lorsque le signal audio indique un événement audio pertinent.

**Fig. 1**

**Fig. 2A**

**Fig. 2B**

**Fig. 3A**

S10 · Z$^{-1}$

Store signal segment — S12

**Fig. 3B**

Transfer signal segment — S22

Denoise — S24

Estimate reliability — S26

Analyze pressure signal record — S28

Update derived data and reliability estimates — S30

**Fig. 3C**

Fill or drain — S42

Check pressure values and event estimates — S44

S46 · $S_P > T_P$ — Yes

No · S48 · $P0-P1 > T_L$ — Yes

No · S50 · $P_P > T_C$ — Yes

Alarm — S52

Safe mode — S54

403 — Net/Svr

UI 401

XTL 410

441

413

414

416

442

448

450

418

402 Osm. agent

404 Electrolyte

433

428

406 Mixing

400

Drain

444

446

Purified
water

431

Water purifier
420

Water

## Fig. 4A

XTL 410

413

414

416

442

448

450

418

402 Osm. agent

404 Electrolyte

428

406 Mixing

400

Drain

444

446

Purified
water

Water purifier
420

Water

## Fig. 4B

403 — Net/Svr

UI 401

XTL 410

413
414
450
418
428
400 →
Drain
416
442
448
441
402 Osm. agent
404 Electrolyte
406 Mixing
444
446
431
Purified water

Water purifier
420

Water

**Fig. 4C**

XTL 410

413
414
450
418
428
400 →
Drain
416
442
448
402 Osm. agent
404 Electrolyte
406 Mixing
444
446
Purified water

Water purifier
420

Water

**Fig. 4D**

**Fig. 4E**

**Fig. 4F**

**Fig. 4G**

**Fig. 4H**

**Fig. 4K**

**Fig. 4J**

**Fig. 5**

**Fig. 6**

900

902    650
       618

904

**Fig. 7**

205  201    202    204   Max rate      206   210   108
                          Max vol

P target —  ⊕  Controller  Final      Pump  Ⓟ
           Σ              controller
                          Drive

207

**Fig. 8**

**Fig. 9**

Time (sec)

**Fig. 10**

Fig. 11

1000

1002

| | | |
|---|---|---|
| **Sensor** 1014 | | **Actuator** 1016 |

**Display** 1018

**Keyboard** 1020

**Mouse** 1022

**Speaker** 1024

**Bus** 1004

**Processor** 1006

**Memory** 1008

**Comm. Interface** 1010

**Network** 1012

**Fig. 12**

**EP 4 061 447 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62938429
- US 2012056781 W
- US 20170203026 A1
- US 20070179422 A
- WO 2007118235 A
- US 2007066251 W
- US 20150005699 A